# EUROPEAN PATENT SPECIFICATION

(11) **EP 0 630 407 B1**
(45) Date of publication and mention of the grant of the patent: **02.08.2000**
(21) Application number: 93905560.4
(22) Date of filing: 10.03.1993
(51) Int. Cl.: C12N 15/62, C12N 15/12, C12N 15/30, C07K 14/445, A61K 38/17

(54) **ANTI-VIRAL FUSION PEPTIDES**
Anti-virale Fusionspeptide
PEPTIDES DE FUSION ANTI-VIRAUX

(30) Priority: 11.03.1992 GB 9205276; 08.07.1992 GB 9214481; 24.07.1992 GB 9215829; 16.09.1992 GB 9219562; 03.03.1993 GB 9304311
(43) Date of publication of application: 28.12.1994
(73) Proprietor: PRENDERGAST, Kenneth, Francis, London SW9 9PE (GB)
(72) Inventor: PRENDERGAST, Kenneth, Francis, London SW9 9PE (GB)
(74) Representative: Holdcroft, James Gerald, Dr.
(86) International application number: GB9300505
(87) International publication number: WO9318160

(56) References cited:
- EP-A- 0 298 280
- WO-A-89/02922
- MOL. BIOCHEM. PARASITOL. vol. 49, no. 2, 1991, ELSEVIER, AMSTERDAM, NY,US; pages 253 - 264 D. NOLTE ET AL. 'A Plasmodium falciparum blood stage antigen highly homologous to the glycophorin binding protein GBP' cited in the application
- PROC. NATL. ACAD SCI. vol. 88, no. 8, 15 April 1991, NATL. ACAD SCI., WASHINGTON,DC,US; pages 3305 - 3309 R.P.TAYLOR ET AL. 'Use of heteropolymeric monoclonal antibodies to attach antigens to the C3b receptor of human erythrocytes: A potential therapeutic treatment' cited in the application

## Description

### FIELD OF INVENTION

The field of this invention relates to protein molecules in particular hybrid protein molecules for the prevention of treatment of human beings afflicted with AIDS and diseases caused by Human Immuno Deficiency Viruses especially types 1 and 2; and other blood borne pathogens.

### BACKGROUND ART

The AIDS syndrome is widely believed by the majority of Scientists (with a few exceptions) to be the end result of infection with Human Immuno Deficiency Virus type 1 or 2. Despite available treatment, on diagnosis of AIDS, life expectancy is short and death occurs usually within two years - death resulting from overwhelming infection and/or neoplastic diseases provoked by a profound immuno deficiency . The causative agent of this condition the HIV virus was proposed by Montagnier L and by Barre-Sinoussi F et al 1983, Science 220, 868-870 and slightly later by Gallo R et al 1983, Science 220 865-867.

The accepted view of most Scientists is that this virus is necessary at least in part for production of this fatal Immuno Deficiency Syndrome. One notable exception to this belief is Deusberg P H who champions the minority view that AIDS is not caused by the Human Immuno Deficiency Virus and that it is not transmissible; Deusberg P H Proceedings National Acad Sci USA Vol 88, 1575-1579, Feb 1991. Regardless of the merits of this contradictory opinion and academic debate, work continues to find methods of combating the HIV virus and removing its presence from infected humans. Since the discovery of the HIV agent in the early 1980's screening of the worlds population has demonstrated that the HIV/AIDS pandemic is much worse than previously imagined. Evidence suggests that the impact of the disease will fall on disadvantaged women and children living in deprived urban areas or in sub-Saharan Africa or the Third World. It is estimated that 10 million persons will contract this condition before the year 2000 Chin J, Lancet 1990; 336: 221-224. Transmission of this virus to newborns from infected mothers remains a growing problem. At the present time in Africa, there are at least 2.5 million infected females who have given birth to an estimated 0.5 million infected infants. Disadvantaged urban areas in the developed world such as areas of New York associated with high rates of drug use have also shown high sero-positivity rates of HIV among neonates ie 2.2% compared with an overall sero-positivity of 1.25% among neonates in that city. NOVAK, Journal of America Medical Association 1989; 261: 1745-1750. The social and economic costs of this epidemic are likely to overburden the resources of health care providers and remains a growing area of concern. Our understanding of the timing of maternal to fetal transmission of HIV has been greatly improved by the study of Ehrnst A et al Lancet. 1981; 338: 203-207. He demonstrated effectively that the virus is transmitted vertically at the time of or close to delivery. Despite high viral titre levels during pregnancy it seems that the mixing of maternal and fetal blood at time of delivery is the most important mode of transmission from mother to fetus. In other contexts viral titre levels are directly related to disease severity; studies by Ho D D "Quantitation of Human Immuno Deficiency Virus type 1 in the blood of infected persons." New England Journal of Medicine 1989; 1621-1625 and the study by Combes R W et al " Plasma Vireama in Human Immuno Deficiency Infections" New England Journal of Medicine 1989; 321: 1626-1631. Both these studies have demonstrated rising levels of plasma vireama throughout the course of HIV and have suggested that high viral titres are associated both with the degree of immuno suppression, the presence or absence of symptoms and the likelihood of tranmission to others. It has been shown that in the early stages of infection, viral titre levels measure 50 virus particles per ml rising to 10-50,000 virus particles per ml or more at the onset of AIDS.

In addition to the offspring of infected mothers, other large groups of the population are at risk or believe themselves to be at risk from the infection, especially homosexuals, drug addicts, persons receiving blood transfusions and health care workers. In the case of health care workers, the risk from a health care procedure carried out on infected persons is likely to be very low and is presently estimated to range of 0.3%-0.5% following needlesticks with an infected needle or surgical instrument. Operating room personnel frequently exposed to needlestick and other injuries are especially concerned at the present time. 8This concern is most marked among Orthopaedic Surgeons who in addition to needlestick injuries are liable to suffer abrasions or even transfixion of a digit with steel wires, pins and the like. So great is the concern that chain-male gloves have been devised to protect staff from inadvertent needlestick injuries. Disadvantages are apparent with barrier methods in relation to HIV prevention. In particular, increased costs - it is known that the dry goods budget of many hospitals has increased considerably following the onset of this epidemic. A second disadvantage is the loss of tactile sensation, making operative procedures more difficult and time consuming. A further problem is the difficulty in attracting and keeping staff in high risk areas who report psychological stress on their part or the part of their spouses, resulting from risk either real or imagined, particularly following needlesticks. A further area of concern is the safety of blood transfusions and the blood supply both in the US and elsewhere. To prevent repeating the iatrogenic infection of large numbers such as haemophiliacs with infected pooled factor 8, there has been a growing awareness of the need to take additional precautions with whole blood and blood product transfusion. The mainstay of transfusion safety remains screening, however no amount of screening can guarantee the safety of a blood transfusion or a blood derived product. Accordingly many workers have devised different methods to treat blood for donation. One is directed to the methods of Sieber US Patent 4, 915, 683 who dislcoses for example photosensatising agents useful to neutralise viruses and other pathogens in blood for tranfusion. This approach and other similar methods is non specific however and requires an irradiating component in addition to a chemical means of treatment.
The present disclosure provides a means of improving the safety of blood products in one step.

The mechanism of HIV infection involves the binding of viral GP120 or envelope peptide to the CD4 peptide molecule. The CD4 protein is a 433 amino acid transmembrane glycoprotein which is found predominantly of the surface of T4 helper cells. It is also found on the surface of the mega karyocytes, monocytes, macrophages and other cells.

The CD4 molecule has three parts: an extracellular part which is divided into 4 variable domains V1 being responsible for the binding of HIV GP120: a short transmembrane region and an intracellular domain.

The CD4 molecule is part of the Immunoglobulin super family. That CD4 was the receptor for the virus was shown by Dalglaoise et al 1984. "The CD4 antigen is an essential component of the receptor for the AIDS retrovirus" Nature; 212: 763-766. Also Madden P J Dalglaoise A G McDougall J S et al "The T4 gene encloses the AIDS virus receptor and is expressed in the immune system and the brain" Cell: 47, 333-348.

Maddon P et al Cell: Vol 47, 333-348 have shown that transformed mouse cells expressing the CD4 molecule possess the capabilities of binding the HIV virus though lack the ability to support the HIV infection at a cytoplasmic level. Debate continues within the art as to the precise binding site of GP120 with the CD4 molecule. Arthus et al Cell: Vol57, 469-491 May 5 1989 proposes that the binding site is contained at least within the first 106 amino acids of the CD4 molecule ie the V1 domain. He bases his assumption on substitute mutants and binding analysis with soluble truncated proteins demonstrating a high binding affinity solely within the first 106 amino acids of the CD4 molecule. Substitution studies led to the notion that the primary binding site on the CD4 molecule lay between amino acids 41-55.

Mizukani et al Proc Nat Acad Sci USA 85; 9273 (1988) and Arthus 1989 "Identification of the residues in human CD4 critical for the binding of HIV" Cell 57; 469 believes that the folded structures of the CD4 molecule brings other amino acids into play. Areas likely to be important in viral CD4 binding are reported to lie between amino acids 41-47 amino acids 16-49, 31-63 and 74-94 of the CD4 molecule.

The remarkable specificity of the virus for the CD4 molecule has lead to the attempts by many workers to utilise CD4 based peptides as therapeutic agents. The binding affinity for the GP120 with CD4 lies in the region of 3nM. Many efforts have been made to utilise this high specificity.

In the case of HIV 2, the affinity is 20-25 fold less, however the binding strength as described is such that even a 25 fold reduction of affinity would not reduce the usefulness of therapeutic molecules based on CD4 and for that reason the binding reaction between GP120 and CD4 remains a logical target for anti HIV pharmaceuticals.

However the nature of the molecule means that a linear peptide will be unlikely to be successful in a therapeutic context.

Brodsky et al Immunology Vol 144, 3078-3086 April 15 1990 No 8 suggests that CD4 based therapeutic molecules should contain amino acids at least from the first 117 amino acids of the CD4 sequence.

Moore J P AIDS 1990, 4: 297-3-5 believes that the CD4 GP120 binding reaction is the one step this virus cannot afford to change by mutation to any appreciable extent.

Accordingly, attempts have been made to use recombinant CD4 peptides as therapeutic agents.

Fisher R et al Nature Vol 331, 7 January 1989, "HIV Infection is blocked in vitro by recombinant soluble CD4" reported the use of recombinant soluble CD4 peptides that showed viral blocking acitivity in vitro. Hussey R et al "A Soluble CD4 protein selectivity inhibits HIV replications and Syncitium formation" Nature 331, 7 January 1989, reported encouragingly that Syncitium formation could be blocked in vitro by the same CD4 based soluble peptides; and Dean et al "Soluble CD4 protein inhibits HIV virus infection" Nature 331, 7 January 1989; A Traunecker et al "Soluble CD4 molecules neutralise Human Immuno Deficiency Virus type 1", also Nature Vol 331, 7 January 1989 encouragingly reports that CD4 peptides may be used as therapeutic agents. The euphoria provided initially by these experiments was short lived.

Problems emerged, in particular the rapid clearance of recombinant soluble CD4 or sCD4 from the blood stream and secondly the existence of multiple binding sites on the virus. It is known that the virus contains no less than a 72 GP120 molecules projecting from its surface. Therefore to fully neutralise a virus particle it would be necessary to combine recombinant soluble CD4 peptides or peptide based molecules with all 72 of the GP120 molecules the kinetics of this event are unfavourable. The present disclosure provides means to overcome both of these problems.
Further discouragement was provided by Eric Daar et al, Proc Nat Acad Sci USA Vol 87, 6574-6578. "High concentrations of recombinant SCD4 are required to neutralise primary immuno deficiency type 1 isolates." This report based on the treatment of 45 patients shows that soluble CD4 peptides failed to consistently reduce HIV titres and further suggests a reason for the failure being lower GP120-sCD4 affinities than being reported in vitro. Daar suggests that mutant strains possessing lower CD4 affinity than the strains used in laboratory testing may be responsible for the failure of the agents.

The present disclosure proposes that clinical disappointment with the T4 agents, such as disclosed in PCT WO89/019140 relates more to the short half life of the peptides in plasma and to the fact that 72 binding sites exist on a single virus, rather than due to lack of affinity between the agent and the virus itself. So great is the affinity between CD4 molecule and the GP120 molecule that even affinities 1000 fold less than those seen in vitro should be pharmaceutically effective.

Despite the failure of unmodified soluble CD4 as a HIV therapy, other workers have advanced alternatives in the hope of utilising the GP120-CD4 interaction. Examples of some of these alternatives are fusion peptides where a CD4 component is fused to another peptide having a different function.

Capon D J et al disclosed the use of CD4 fused to the FC component of the human Igh antibodies. The objective being to mobilise the compliment system and thereby destroy the virus particle by the compliment cascade. Capon refers to these new molecules as immuno adhesives. A possible disadvantage associated with this approach is the necessity of a fully functional compliment system, unlikely to the case in advanced HIV infections. See Capon et al "Designing CD4 Immunoadhesins for AIDS Therapy" Nature Vol 337.

The molecular machines of the present disclosure do not require a functional immune system and may be effective in cases where no immune function exists.

At the present time the main stay of treatment relies of the substance Azidothymine or AZT, this susbtance acts on the HIV enzyme reverse transcriptase.

An increasing number of patients and earlier in their illness are receiving treatment with AZT, however tolerance of this drug can be a problem. AZT is known to be lymphotoxic and is additionally associated with substantial anaemia. Haemato toxicity appears to be major limiting factor in the use of AZT in the treatment of ARC and AIDS Richman et al "The toxicity of the Azidothymine, New England of Medicine, 317; p 192-197, 1987. So profound is the anaemia provoked by this agent that many patients require frequent blood transfusions or treatment with Epogen (R), a novel genetically engineered erythropotein. The molecular machines of the present disclosure may be used in combination with AZT or other treatments and will augment the effectiveness of blood transfusions given to patients receiving AZT who are suffering from severe anaemia. Other agents acting on the reverse transscriptase such as DDC or DDI suffer high side effect profiles also and are toxic compounds in their own right. Equally, agents acting at other sites such as glycosylation are known to exhibit severe toxic effects on the host.

The agents of the present disclosures are anticipitated to have lower toxic effects on the host than existing treatment modalities.

The agents may be used alone or in combination with existing treatments.

So great is the side effect problem of anti-AIDS therapeutics many feel it is likely that no one agent will be successful in combating this disease. One is directed to Fisher World Patent WO 89/11860 for a discussion of combination therapy.

More recently Taylor R P in Proceedings National Acad Science 88, p 3305-3309 refocused our attention on the work of Nelson published in 1950's who some years ago proposed an imune function for the red cells. Red cells (erythrocytes) are often considered to have only oxygen carrying and acid base balancing functions in the blood stream. According to Nelson red blood cells have additional immune functions. In particular Nelson proposed that opsonised antigens ie antigens whose surfaces were bound to compliment derived peptides could be fused to the compliment 1 receptor molecule (CR1) on the red blood cell surface. By fusing a virus or bacterium to the red cell surface in this way the virus or antigen becomes bound to the red blood cell surface in the manner of a barnacle bound to the surface of a ship.

A virus immobilised in this way would be harmless and be carried around the circulation on the red cell surface to await final phagocytosis and digestion at the end of the red cells life (usually 120 days from formation). It is thought that many antigens and pathogens are cleared from circulation by this mechanism rather than by direct phagocytosis and the like so frequently discussed in immunology texts. Accordingly, Taylor R P proposed a technique to utilise this long forgotten mechanism. He suggested the use of hybrid antibodies capable of binding both to the HIV virus and to the compliment 1 receptor molecule of the red cell surface. The adherence of virus particles to the red blood cell surface membrane in this way would clear the blood stream of free virus particles and free GP120. Thereby attempting to achieve Nelson's proposition that one day red blood cells could be harnessed to rid mankind of infection.

The peptide machines of the present disclosure provide totally different mechanisms to achieve Nelson's dream ie to bring about the binding of HIV virus particles to the red blood cell surface membrane.

The agents of the present disclosure carry the additional advantage in that antibodies to the compliment peptides and red blood cell surface CR1 are not a feature. This would be an advantage in that the risk of destabilising an already unbalanced immune system is not expected to be a feature of the molecules of the present disclosure.

The Third World or underdeveloped world is no stranger to epidemics and has prior to the development of AIDS and HIV been ravaged by infectious disease: the most serious on a global scale is probably malaria. Various forms of importance to humans are Plasmodium Falciparum, Plasmodium Vivax, Plasmodium Ovale, Plasmodium Malariae. This serious parasite infects red blood cells. The life cycle is complex and consists in part of a short life cycle in the salivary gland of mosquitoes. Following innoculation into a human being, the malaria organism migrates to the liver and to the red blood cell. It multiplies forming further merozoites. Merozoites may then proceed to infect other red blood cells, or may find their way into another mosquito where they replicate in the mosquito salivary gland and later proceed to infect another human being.

The course of malaria is a variable one and may be characterised by a short acute illness which can bring death in a matter of hours; or a longer more chronic illness associated with debility and anaemia. Other forms of malaria infect animals such as plasmodium Knowlesi which is known to infect the Rhesus monkey: and many other zooanotic forms.

The preferred location for the malaria parasite is within the red blood cells of the infected host and for much of its life span it lives intracellularly protected from the host immune system.

Merozoites are thought to spend but a brief period free in the circulation. Accordingly, considerable research efforts have been expended to discover the means whereby the merozoite forms of the parasite gains attachment and gains entry into the human red blood cell.

Margaret E Perkins Journal Experimental Medicine 160 September 1984, 788-798 is responsible for the formulation of a relationship between the Plasmodium Falciparum glycophorin binding molecule and glycophorin A and B two sialo glycoproteins found on the surface of red blood cells.

More laterally, Holt and Perkins et al American Journal Tropical Medicine Hygiene 1989, p 245-251 disclose species and stain variations of Plasmodium Falciparum wherein some strains of the organism exhibit preference for sialo glycoproteins glycophorins A, B and C; and also demonstrated was the varying requirements for the N-acetyl-neuramic acid residues (NeuNac).

The glycophorin A molecule is a highly glycolated peptide. Pasvol has suggested that glycophorin binding peptide binds to the region of glycophorin close to the lipid bi-layer. Pasvol G et al "Inhibition of Malaria Parasite Invasion by Monoclonical antibodies against glycophorin A correlates with a reduction in red blood cell membrane deformality." Blood 74, No 5, Oct 1985, 1836-1843. Debate continues within the literature as to the requirement for sialic acid on the glycophorin molecule to effect invasion by merozoites.

Some strains of malaria are totally dependent on normally sialated glycophorin A to gain entry into the red cell, whereas other strains seem to be independent of sialic acid. See Mitchell et al 67, No 5 May 1986 1519-1521. Perkins and Roco in Journal of Immunology, 88, Vol 141, 3190-3196 No 9, again stress the importance of sialic acid where normally silated glycophorin is necessary to achieve successful binding of merozoite peptides, in particular to Pf200.

For several years a peptide called the glycophorin binding protein was believed to be the primary peptide responsible for binding merozoites to erythrocytes. A gene coding for GBP was isloated by M Ravetch and Kochan J and disclosed in Science Vol 227, p 1593-1596, 29 March 1985 and incorportated herein fully by reference.

GBP 130 is characterised by a tandem repeated sequence believed to be the site of erythrocyte binding, "A tandem repeated sequence determines the binding domain for an erythrocyte receptor binding protein of plasmodium falciparum". Kochan J, Perkins M and Ravetch J Cell, Vol 44, 689-696 March 1986. See figure 2 p 691 which also discloses the full sequences and genetic code of the GBP 130 molecule and is incorporated fully herein by reference.

Other workers have challenged the supremacy of the GBP 130 as the primary binding molecule of the malaria merozoite, Orlandi P; Kim Lee Sim et al Molecular and Biochemical Parasitology, 40 (1990) 285-294 "Characterisation of the 175 kilodalton erythrocyte binding antigen of plasmodium flaciparum" suggested a different peptide, the EBA 175 molecule, as being responsible for merozoite binding or at least playing some role therein. The EBA 175 molecule like the GBP 130 molecule has an affinity for the red blood cell surface and binds thereto.

It is known that the EBA 175 molecule has an affinity for oligosaccharides which are found on the surface of the red blood cell. However a problem arises in that the EBA 175 molecule does not bind effectively with the malaria merozoite parasite. Therefore it is thought that the EBA 175 serves a function as a bridge. This disclosure proposes an alternative mechanism in that the EBA 175 molecule is responsible for bringing the merozoite close to the erythrocyte membrane surface, thereafter GBP 130 drags the merozoite closer still by binding with the base of the glycophorin A peptide; thus bringing the lipid bilayer of the malaria parasite into approximation with the lipid bilayer of the red cell membrane and thereby allowing the incorportation of the parasite into the erythrocyte itself. This disclosure suggests the merozoite is winched into the RBC cytoplasm.

The genetic sequence and the peptide sequence of EBA 175 was disclosed by Kim Lee Sim, Orlandi P et al "Primary structure of the 175 K plasmodium falciparum erythrocyte binding antigen and identification of a peptide which elicits antibodies that inhibit malaria merozoite invasion See J Cell Biology Vol 111 (1990) p 1877-1884 figure 2 of P 1880 for the sequence of amino acids and DNA sequence.

To further complicate the picture Dagmar Nolte et al described two close relatives of the glycophorin binding peptide 130 molecules which they call GBPH or glycophorin binding peptide homologues. These molecules like the GBP molecule display several tandem repeat sequences and a high affinity for the erythrocyte surface membrane surface peptides. It has been proposed by Nolte and co-workers that it is the GBPH molecule and not the GBP molecule that is responsible for erythrocyte binding of the parasite in that the GBP molecule is released as an immunogenic decoy to distract the immune system from the real binding peptide the GBPH.

The nucleotide sequence and amino acid sequence of one form of the peptide GPBH is dislcosed by Dagmar Nolte et al in the Journal of Molecular and Biochemical Parasitology 49, (1991) p 253-264 see figure 2 of p 257 incorporated herein fully by reference. See also figure 3 of p 258 the same journal and paper which lists a comparison between GBP 130 and GBPH. Binding and entry of merozoites into RBC's probably involves several peptides or several alternatives as fail safes for the organism.

The picture is further complicated by other research notably by Peterson Gregory who proposes PMMSA (Pre major merozoite surface antigen) as being responsible for erythrocyte binding either in this state or following fragmentation into smaller fragments. The genetic sequence and the peptide sequence of the PMMSA molecule is given in the Journal of Molecular and Biochemical Parasitology 27 (1988) 291-302 see figure 3 of P 294 and 295 Peterson G et al, and is incorporated fully herein by reference.

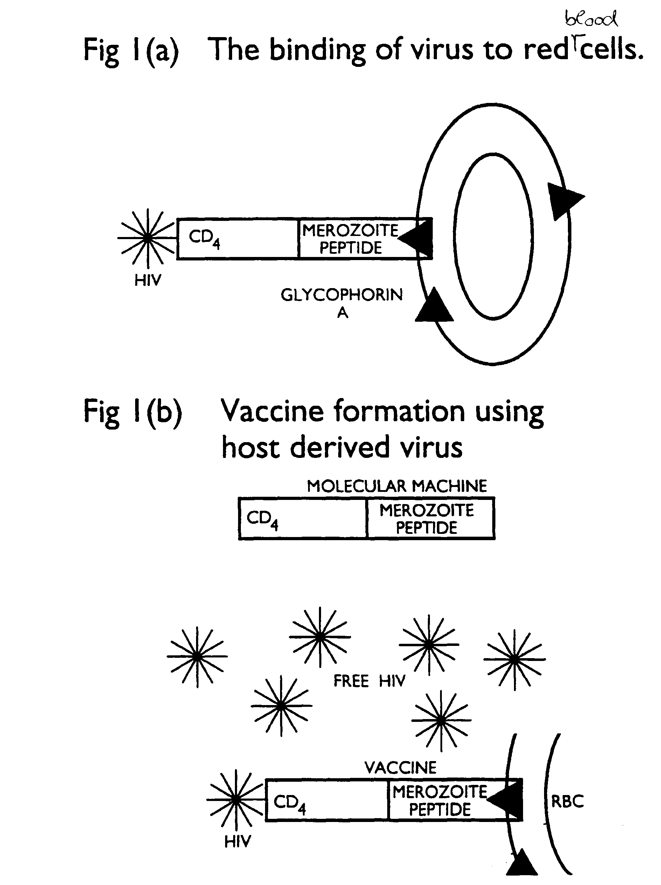

Erythrocyte binding using different peptides and surface molecules is exhibited by other species of the malaria parasite in particular the Plasmodium Vivax organism. This organism can infect only persons expressing the Duffy marker. The Duffy antigen is a red cell surface marker and is one of many blood group markers and carried by a percentage of the population. Persons not expressing Duffy antigens are therefore immune from infection by Plasmodium Vivax. The Plasmodium Vivax merozoite expresses a Duffy binding receptor molecule the P. vivax Duffy receptor, cloned and sequenced by Xiangdang Fang and disclosed in Molecular and Biochemical Parasitology 44 (1991) p 125-132 see especially figure 1 of p 127 for the genetic sequence and amino acid sequence. Similar to Plasmodium Vivax is Pladmodium Knowlesi which also uses the Duffy antigen. This organism parasitises Rhesus monkeys. Also in the same Journal, same figure, same page is listed the genetic sequence of Plasmodium Knowlesi Duffy receptor molecule which may find a use in the machines of the present disclosure.

When developing therapeutic agents directed against the malaria parasite itself then it is the teachings of the art to identify the precise molecule responsible for merozoite binding in the clinical context. However, this disclosure teaches that where malaria peptides are to be employed as erythrocyte binding agents more generally then it is not important to identify the precise peptide the malaria organism uses to effect invasion, rather any malaria peptide capable of binding to an erythrocyte surface membrane may have a therapeutic use for the purposes of the machines of the present disclosure and also segments of such a peptide.
It is disclosed that the machines of the present invention consists of hybrid peptides formed by the fusion of malaria derived peptide sequences sufficient to exhibit erythrocyte membrane affinity fused to a CD4 derived peptide sequence sufficient to demonstrate affinity for the HIV virus. The restriction map and peptide sequence of the CD4 molecule are disclosed by Madden et al Proceedings National Acad Science USA 84: 1987' p 1955-1959 see especially figures 1 and 2 incorporated herein fully by reference.

Also Madden et al Cell Vol 42, P 93-104 August 1985 especially figure 6 of P 97.

It will be appreciated that the CD4 peptide components will not be restricted to segments of the naturally occurring CD4 molecule. Also, the malaria derived peptide fragments may be any that are capable of binding to the red cell membrane and one is especially directed to those referenced herein before.

### BRIEF SUMMARY

The present disclosure provides novel hybrid or fusion peptides having a minimum of two different peptide components possessing different functionality, according to claim 1. One component, preferably the CD4 molecule or segment or derivative thereof which is capable of binding to an HIV virus is fused or joined to a malaria derived peptide or derivative or fragment thereof which is capable of binding to a red cell membrane. The fusion peptide will in one aspect bind the free HIV virus to a red cell producing possible therapeutics benefits of:
a) reduced free virus in circulation;
b) reduced free GP120 in circulation:
c) reduced symptoms and infectivity, both generally and prior to delivery;
d) augmented therapeutic usefulness of blood tranfusions;
e) greater safety of blood tranfusions:
f) immune augmentation by novel vaccine effects;
g) usefulness as prophylaxis, eye drops for theatre staff; preparations, creams, lotions and foams for contraceptive use; preparations for exposed cuts or abrasions;
h) simpler safer and economic viral titre measurements;
i) preparation for cleaning surgical instruments.

It will be appreciated that more than the minimum two components may be used to form the fusion peptides,mixed in any combination to produce more complex fusion peptides without departing from the scope and spirit of the invention.
eg 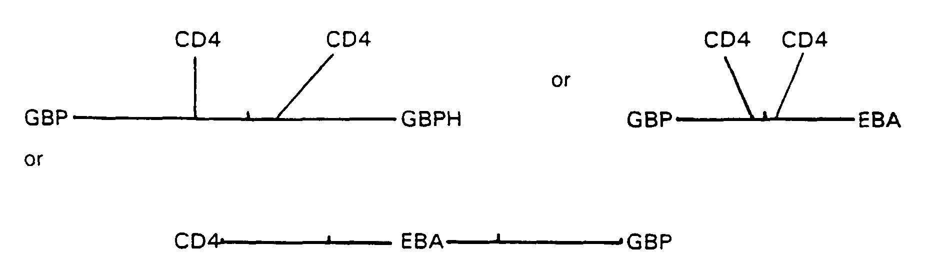
CD4 CD4
or
CD4 CD4
GBP GBPH
GBP EBA
or
CD4 EBA GBP
 or where A denotes a malaria fragment and B denotes a CD4 derived fragment and 'm' and 'n' are natural numbers, the disclosure contemplates in some aspects the hybrid molecules such as:-
Am Bn; (ABA)n
(AB) n; (BAB) m
and the like which also fall with the scope and spirit of the present disclosure.

### DESCRIPTION OF DRAWINGS

The figures 1(a) and 1(b) are provided on Drawings sheet 1 of 1.
Figure 1(a) depicts free HIV virus particles drawn to and immobilised on a red blood cell (RBC).
Figure 1(b) depicts the formation of a novel vaccine within the host blood stream utilising HIV components (live virus) derived from the host rather than provided from external sources as would be usual; for vaccines.

### DETAILED DISCLOSURE AND EXEMPLARY EMBODIMENTS

The fusion peptides of the present disclosure are prepared by the conjugation of at least two separate components into a single larger molecular entity or conjugate peptide. A minimum of one HIV binding component is joined to a minimum of one RBC peptide. A minimum of one HIV binding component is joined to a minimum of one RBC binding component. The components may be joined directly or indirectly to a junctional sequence or cross linkers.

The simplest form of a fusion peptide will consist of a HIV binding peptide and an RBC binding peptide fused by peptide bonds giving the following structure:

HIV Binding
Peptide
RBC Binding
Peptide
Middle of
molecule & site
of fusion not
critical

The functional areas of the machines are preferably situated towards either end. Therefore the middle of the molecule and the site of fusion is not critical.

It will be appreciated that the two components may be fused directly or an intervening segment or peptide sequence or linker be interposed between them. Such a segment could be any peptide sequence such as:
a) the junctional areas of the CD4 molecule;
b) the Fc segment of an immunoglobulin;
c) or any macromolecule or cross linker or part thereof.

The interposed segment may or may not retain functionally of its own but must not interfere with the function of HIV binding and RBC binding. The preferred HIV binding component is a CD4 peptide sequence all or part thereof retaining HIV binding. However, it will be apparent to the skilled artisan that a CD4 therapeutic agent such as CD4-Fc retaining a funtional HIV binding moiety would also provide a suitable HIV binding component. In that event, the Fc fragment would become a spacer or peptide cross linker between the CD4 segment and the RBC binding peptide.

The alternative becomes:

CD4 | SPACER | RBC BINDING PEPTIDE
PEPTIDE
CROSS LINKER

It will be appreciated that the use of a CD4-Fc peptide or any CD4- P where P is a non specific peptide sequence, in place of a simple CD4 peptide segment falls, within the scope and spirit of this invention regardless of whether the middle component retains functionality or not. One is directed to WO 89/02922 Genetech Danial J Capon for a description of immunoadehesions comprising eg CD4-Fc fusion peptides which may be used in place of CD4; and is incorporated fully herein by reference.

The envisaged mode of action of the therapeutic fusion peptides of the present disclosure is that one component of the molecular machines, the CD4 derived component will bind with the HIV virus GP120 molecule or free GP120 whilst the merozoite derived peptide segment will bind with the erythrocyte membrane surface. Either glycophorin A, B, C or to the Duffy antigen where a Duffy receptor peptide is used.

The outcome of this binding will be to immobilise the virus particle onto the surface of the red blood cell much like a barnacle to the surface of a ship, and thereby neutralise it.

Thereafter the virus will be carried around with the red cell harmlessly to await destruction by mechanisms such as phagocytosis.

It is likely that the peptides will have a long half life in vivo. It is known that merozoites have a predilection for young red cells rather than mature red cells. Therefore the problem of short half life seen with previous CD4 based therapies will be eliminated to a certain extent. The effect of this viral binding will be to sweep the circulation clear of free virus particles, reducing the victims infectiveness to others. The peptides of the present disclosure will find uses as molecules to be injected prior to surgery, molecules to be injected prior to delivery, molecules to be mixed with blood transfusions (whole blood) to be given to AIDS victims thereby to improve the therapeutic effectiveness of tranfusions and molecules to reduce the high viral titre levels believed to be associated with symptomatic AIDS and molecules for prophylaxis generally.

A further benefit of the machines of the present disclosure is the possibility of incorporation of the virus into the red blood cell cytoplasm. It will be appreciated that although this step is not essential to the present disclosure, immobilisation being sufficient, nevertheless were a virus to attempt infection of a red cell such infection resulting in the dismantling of the virus outer coat and the incorporation of the virus RNA into the red cell cytoplasm this would be a completely wasted exercise from the viral point of view. Red blood cells lack a nucleus and a virus once incorporated into a red blood cell cytoplasm is immobilised and neutralised forever.

It is envisaged that normal dosage levels of a peptides of the present disclosure will range from 0.01 mg/kg to 10 mg/kg. The agents of the present disclosure may also find a use in diagnostic testing and viral titre quantification.

The molecular machines of the present disclosure may act beneficially in further ways.

The GP120 binding component (CD4 derived sequence) may bind the molecular machines to infected cells expressing GP120. Expression of GP120 by infected cells occurs prior to viral shedding

The resultant complex, 
infected
Cell——— surface GP120 ——— CD4 ——— malaria peptide
 may mobilise an immune reaction against the infected cell killing the infected cell thus preventing viral release.

Further were this immune response to be in the form of cellular immunity or continue in the absence of the molecular machines of the present disclosure, then the said machines would constitute a long awaited vaccine or immuno modulator against HIV.

It is known for instance that cellular immunity is highly desirable in combating HIV infection. It is known that HIV victims with enhanced cellular immunity live longer and remain symptom free for long periods.

The generation of a cellular immune response has been found to follow where an infection antigen (virus) has been ingested by a macrophage APC (antigen presenting cell). Live viruses are processed differently to non infectious particles by APC's. Live viral antigens are digested in the cytoplasm of the APC (cytoplasmic pathway) and digested particles presented on the APC surface in association with class 1 MHC markers provoke a cellular immune response (Whitton J L Oldstone M B A Class 1 MHC can present endogenous peptide to cytotoxic lymphocytes J Exp Med 1989 170: 1051-56). Accordingly the degradation of HIV (live virus) coated RBC's in the spleen or reticulo endothelial system could encourage a cellular immune response. By capturing endogenous live virus and by binding the said live virus to a cell destined for destruction by phogocytosis, the fusion peptides of this invention provide a vaccine of a particularly novel variety in that:
1) the vaccine as administered contains no components derived from the pathogen target of the vaccine (no HIV component). eg rGP120;
2) the vaccine obtains its pathogen derived component direct from the host and modifies host produced live virus;
3) the red blood cell surface is incorporated into the final immunogenic construct.

It should be noted that the art teaches that vaccines should usually contain material derived from the target pathogen. This disclosure teaches vaccines which do not provide target pathogen-derived material in that the material derived from the target pathogen of the vaccine is provided later in the host. The precise mechanism for the generation of cellular immunity is not understood. It is a further unproven postulate of this disclosure that cellular immunity generally proceeds from the processing of pathogens bound to erythrocytes when the said pathogens are engulfed with their red cell symbiot in the liver, bone marrow or spleen. Possible proof of this hypothesis lie in the following facts:
1) the fact that splenectomy leads to a defect in cellular immunity;
2) the fact the splenectomy is beneficial in hypoimmune states such as ITP idiopathic thrombocytopaenic purpurpae.
3) The fact that cellular immunity is a delayed phenomenon which would be consistent in that the spleen must wait for RBC's with bound antigens to 'age' sufficiently before engulfing them.

The precise role of the spleen in the 'immune' system is not clearly understood.

However, although it is useful to speculate on the above hypothesis they are disclosed herein as notions worthy of further study, and nor binding to the disclosure.

### EXAMPLES OF CLINICAL MODE OF USE OF MOLECULAR MACHINES

### EXAMPLE 1 OF CLINICAL MODE OF USE

The fusion peptides of the present disclosure in one aspect are prepared and packaged in lypholised form and stored in refridgerated conditions. The lypholised fusion peptides are reconstituted by means of injectable solvents such as injectable preparations of normal saline or dextrose 5% or the like together with buffers and other stabilising ingredients incorporated as necessary.

The reconstituted fusion peptides are administered to patients by either the Direct or Indirect method.

Direct method involves the intravenous or even intra muscular administration of the reconstituted injectable solution of the molecular machines of amounts usually not exceeding 1,000 mg of agent per ml. The agents may be infused slowly by means of an intravenous drip or when safe to do so or if necessary by bolus injection.

The slower administration rates provide more even mixing of agent with red cells therefore labelling many more red cells.

A disadvantage with targetting large numbers of red cells is the risk of haemolysis. But the advantage is longer half life of the agent. Bolus administration or rapid administration reduces haemolysis because fewer red cells are carrying the agents, however those fewer red cells will have a shorter plasma half life.

Indirect methods of administration envisage the mixing of the reconstituted agents with red cells outisde the body. The agents may be added to blood transfusion units all or part prior to administration or the agents may be mixed with a quantity of the patients own blood. Ten mls of the patients own blood is mixed with citrated buffer and thereafter with the reconstituted molecular machines. A degree of mechanical agitation is provided. The treated blood is now reintroduced into a suitable vein, slowly at first to observe for untoward reactions.

The procedures above may be repeated until side effects such as haemolysis or idiosyncratic reactions prevent their further use. It is envisaged that the agent will be administered as courses of treatment on a 3 to 4 monthly basis.

It will be appreciated that the agents are not necessarily restricted to lypholised forms. It has recently been shown that following liquid storage recombinant peptides perform better than freeze dried preparations, in some cases this was demonstrated for IFN see Pearlamn and Tue Nguyen J Pharm Phamacol 1992, 44 (suppl 1): 178-185.

### EXAMPLE 2

The agents may be formulated in slow release forms allowing the gradual leaching of active peptide molecular machines over a time period. Examples of such methods are the incorporation of the agents into biodegradable polymers. Such polymers could be injected as subdermal 'rice grains' or form part of an intrauterine contraceptive device.

Intrauterine contraceptive devices (IUD'S) impregnated with the agents of the present disclosure would slowly leak the agents towards the cervix. Cerival erosions are known to be an area of major importance in women's vulnerability to AIDS. Indeed the heavier menses associated with IUD's would leach more of the peptides from the polymer and by targetting menstrual blood or on their own provide a useful degree of protection against the virus. Such a use would be very helpful in the Third World.

For a useful reference on polymer delivery systems see Kost J and Langer R Tibech April 1992 Vol 10, 127-131 incorportated fully herein by reference.

### EXAMPLE 3

The novel peptide agents of the present disclosure may by incorporated into creams and gels or foams for use as topical preparations. Such compositions may be used in conjunction with:
a) barrier contraceptives;
b) in place of existing lubricating agents or with lubricating agents generally

The said creams, gels or foams may also be used to dress wounds, cuts or open sores or as contraceptive agents or in conjunction with contraceptives especially barrier methods. In the case of HIV positive patients such sores would become reduced sources of infection for others.

In the case of medical personnel such creams, gels or foams and the like could render abrasions or cuts more protected against contamination by free virus from patients. Such a mode of use as a prophylactic may be especially helpful for theatre personnel.

### EXAMPLE 4

The agents of the present disclosure are formulated into drops, especially eyedrops. During surgical operations eye splashes are a frequent occurrence. Theatre staff although strongly warned to use eye protection often fail to do so for reasons of discomfort, haste or carelessness. Existing eyewash preaprations do not contain specific anti-viral components. The present agents are formulated as surgical eyedrops or eye washes useful as emergency decontaminants of splashed eyes during operative procedures.

### EXAMPLES OF CLINICAL USE AS TESTING AGENTS

### The liquid phase use of malaria erythrocyte binding peptides fused to CD4 in HIV tests and viral titre quantification:

A measured volume of blood or fluid to be assayed is mixed with a known quantity of molecular machines; usually 1 ml of sample blood with 20 ug of CD4-merozoite fusion peptides. following a period of incubation, agitating the sample so as to bring red cells into contact with the virus, the sample is further incubated with a fluorescent labelled anti HIV GP120, anti GP160 or anti GP41 antibody or cocktail thereof. Separation is carried out by centrifugation or filtration. After adequate washing of cells by appropriate buffer, cell fluorescence is measured in a cell sorter. The advantages of such a liquid phase HIV test are:
1) high specificity owing to the concentration of antigen in one small spot on a red cell, helping fluorescent contrast
2) ease of automation
3) quantification of viraemia. Up to now HIV viraemia has been difficult to quanity. Cheaper methods of viral quanitification will assist in new drug development.

### EXAMPLES OF METHODS OF MANUFACTURE

The fusion peptides of the present disclosure may be manufactured by a wide variety of manufacturing methods and strategies: which may be applied in various combinations.

Both the number and variety of suitable methods of manufacture are increasing rapidly at the present time and are widely known and practiced by persons skilled in the art. Accordingly in the interests of brevity reference will be made to some but by no means to all of the manufacturing methods suited to the present disclosure.

The various manufacturing methods may be combined in different combinations to form a final strategy. In one strategy the fusion peptides may be manufactured by means of the separate manufacture of components such as components A and B and subsequent fusion of A and B invitro using various means eg by chemical linkers. Peptide sequences even greater than 150 amino aicds long may be manufactured entirely synthetically invitro. Accordingly where component A or B of the fusion peptide is less than 150 amino acids in length this component may be made synthetically as a single component. Where component A or B is very long component A or B may be manufactured synthetically in smaller fragments to be bonded chemically there after invitro, the said bonding to be preferably peptide bonds and achieved by methods such as segment condensation or by other methods known to the art.

The two peptide components A and B may also be fused using chemical linkers or by disulphide bonds as disclosed by Chan et al 1977, J Bio Chem 252; 1515-1522. Miskimins et al 1979 Bio Chemi Biophys Ref Comune 1991; 143-151 discloses a fusion peptide formed using cystamine as the cross linking agent. Cawley et al 1980 Cell 22, 563-70 discloses the use or hetrobifunctional cross linking agents to form a hybrid molecule consisting of RlClN A joined to epiderminal groth factor EGF. Other useful strategies for fusing peptide sequences employ linking groups such as the chelating agent DTPA. To facilitate the fusion of peptide sequences by means of chemical linking groups it may be helpful to fix one or both peptide sequences onto a solid substrate such as resin beads or polyacrylamide gel or cross linked polystyrine or other anchoring mority.

The recent art continues to provide new techniques for applying the Merrifield synthesis of peptides on a scaled up basis useful for the large scale manufacture ie of peptides such techniques are termed "scale up of solid phase peptide synthesis. Merrifield synthesis may be very suited to the tandem repeat segements of malaria derived peptide components. In the interests of brevity a full account of peptide synthesis will not be given which techniques are widely known and practised by the skilled artisans but reference will be made to some more interesting or recent developments in peptide manufacture.

A recent development involves the use of a new anchoring moiety involving the bondying of (HYCRAM) (R) (Orpegen GMBh, Czernyring 22, D-6900 Heidelberg FRG) a 4-hydroxy-crotonoyl-amidomethyl group bonded to an aminomethyl-polyacrylamide gel via spacer molecules such as a B-alanaine or sarcosine or the like. Fmoc-amino acids can be linked to the HYCRAM (R) by esterification. Also any Boc-amino acid or any Ddz-amino acid 3,5-di methoxy phenyl-2-propyl-2-oxycarbanyl-amino acid salt may be bonded to the HYCRAM (R) anchor.

Synthesis then proceeds using the Boc-/benzyl, the Ddz-/t-butyl or the Fmoc-/t-butyl protocols as usual.

Detachment of the peptide from the HYCRAM (R) support employs palladium tatra-kis (triphynyl-phosphane) a catalyst in a suitable solvent such as 50% (v/v) dimethylsuphoxide with dimethyl formamide: N-methylpyrrolidine, tetrahydrofuran and water. Oxygen tetrahydrofuran must be excluded. Acceptor molecules, morpholine, dimedine or N, N'-di methylbarbiturate may be added to take up the allylic group.

The Ddz-/t-butyl amino acid protections are easier to cleave using with 1-5% (v/v) trifluroacetic acid in dichloromethane a process taking 10 to 30 minutes or by means of the more environmental friendly acetic acid or dioxane containing 1 % (w/v) HCL gas. The other useful protocol is the Fmoc-/t-butyl strategy. Cleavage of F moc can be achieved using 20-50% (v/v) piperidine/dimethyl formamide. Deprotection can be monitored in both cases photometrically. The activation of Boc-; Fmoc-; or Ddz-amino acid derivatives may employ the inexpensive (DCC) diclohexylcarbodi-imide. Pre activation using HOBT (N-hydroxybenzotriazole) can be employed to form symmetric anyhydrides of protected amino acids or their esters. Other activating agents are the Castro Reagent or BOP: Benzotriazole-l-yl-oxy-tris (dimethyl amino) phosphonium hexa fluorophosphate; one is directed to CASTRO B et al (1957) Tetrahedron Lett 14, 1219: and TBTU the Knorr reagent, Benzotriazole-l-yl-oxy-l, 1, 3, 3-tetramethyluronium tetrafluoroborate one is directed to Knorr R et al (1989) Tetrahedron Lett 30, 1927. Fragment condensation can be achieved using the BOP or the TBTU reagent with HOBT in excess. Protected peptides may also be in excess, however solvents and excesses can often be recycled.

It will be appreciated that by blocking incomplete fragment condensations shorter by-products can be discriminated from the desired polypeptide. Using this system high purity polypeptides can be produced.

Monitoring of the production process will usually involve U.V. absorption techniques.

A typical production process involves either the separate synthesis of peptide sequences by their expression in suitable hosts, and their subsequent purification and fusion; or chemical synthesis such as on a solid substrate for example by the sequential addition of amino acid residues or peptide fragments which are protected, the protection of the amino acid residues as required and the subsequent reacting of the peptide chains with linking agents before removing the peptide chains from the said solid substrates and the final purification by the various means is such as reverese phase chromatography: or any combination of the above.

In the case of some of the exemplary embodiments it may be convenient to manufacture the fusion peptides by means of a fused gene. A fused gene is a genetic sequence which codes for both components of the hybrid component molecule. One is directed to Murphy United States Patent 4,675,382 for a detailed disclosure of the use of fused genes in the manufacture of hybrid peptides having the components MSH or Melanocyte Stimulating Hormone fused to dipcheria A toxins.

Alternatively peptide fragments may be manufactured by DNA cloning and expression in suitable hosts and recovery with subsequent condensation in vitro.

Generally cloned sequences useful for the production of fusion peptides will have the transmembrane domain and the cytoplasmic domain sequences removed.

For a useful general description of DNA cloning and modular hydridization technology, one is directed to Mainiatis et al Molecular cloning. A Laboratory Manual, Cold Spring Harbour lanoratory (See Second Edition 1989); and to Horvath et al An Automated DNA synthesiser employing Deoxynucleoide 3 phorphoramidites, methods in enzymology 154: 313-326, 1987. One is also directed to "Principles of Gene Manipulation an Introduction to Genetic Engineering" Old RW and Primrose S B Ed 4; Blackwell Scientific Publications ISBN 0-632-02608-1 incorporated fully herein by reference. DNA may be made by the chemical synthesis of DNA polymer fragments using phosphotriester, phosphite or phosphoramidite chemistry. For a description of solid phase techniques one is directed to Chemical and Enzymatic Synthesis of Gene Fragments - A Laboratory manual ed H G Gassen and L Lang, Verlag Chemiee, Weinheim 1982; and Gait M J et al Nucleic Acids Research 1982, 10, 6243; Spoat B S et al Tetrehedron Letters 1980, 21, 719; Matteuci M D et al J of American Chemical Society 1981, 103, 3185; Sinha N D et al Nucleic Acids Research 1984, 12, 4539; Adams S P et al J American Chemical Society 1983, 195; 661; and Matthes H W D et al Embro Journal 1984 , 3, 801; and are incorporated herein fully by reference.

Reverse transceptase techniques may also be used to generate a complimentary c DNA strand by means of the reverse transciption of malaria parasite derived mRNA. Kits are available for this purpose.

The DNA fragments may be ligated by either blunt-ended or staggered-ended termini using restriction enzymes; digestion; filling in as required; and treatment with alkali and phosphatase for protection and subsequent ligation with suitable ligases.

Appropriate leader sequences may be chosen from the many available.

The cloning of the DNA sequences of the hybrid peptides of this invention may take place in prokaryotes such as E coli for example K12 strain 294 or E coli B or E coli X1776 by way of non limiting examples or by means of the polymerase chain reaction.

Subseqent expression of the hybrid peptides may take place in any host cell, but preferably mammalian host cells. Other useful cells are fungi, yeasts, insects and prokaryotes. Signals suited to the chosen host cell are chosen as appropriate, in the case of prokaryotes one can chose from a large group including alkaline phosphatase and the like.

Where prokaryotes are used to express the hybrid peptides, then they are transformed by an expression vector usually a plasmid such as PBR322 into which the DNA encoding the fusion peptide or fragment has been ligated such a plasmid will also feature suitable marker sequences, promoters and Shine-Dalgarno sequences may be chosen as appropriate.

A prokaryote host such as E coli may be transformed by treatment using a solution of CaCl₂ as described by Cohen et al PNAS 1973, 69: 2110 or by treatment with a solution comprising a mixture of RbCl, Mncl₂, potassium acetate and glycerol and then subsequently with 3 - (N-morpholino) - propene-sulphonic acid and RbCl and glycerol.

One is directed to "DNA Cloning" Vol II D M Glover ed, IRL Press Ltd 1985 for a description of transforming techniques.

Alternatively where the chosen host is a yeast such as Saccharomyces cerevisiae the plasmid YRp7 as the expression vector may be used. One is directed to Stinchcomb et al Nature 282, 39, (1979), Kingsman et al Gene 7; 141 (1979); Tschemper et al, Gene 10; 175 (1980).

On some occasions difficulties were encountered in cloning segments of the plasmodium falciparum DNA resulting from the very rich A T DNA sequences which cause problems when cloned in E coli organisms.

Using yeasts such as saccharoyces cerevisiae large segements such as 230 kilobases may be cloned successfully using yeast artificial chromosomes such as pYAC-4; Tony Triglia and David Kemp, Molecular and Biochemical Parasitology, 44, 1991, 207-212. As has mentioned herein before the cloning of soluble of T4 peptides has already been achieved and represents little difficulty. One is directed to PCT Patent Application WO 89/11860 and to US Patent Application 094322 filed Sept 4 1987 and to US Patent Application 141649 filed January 1987 and the PCT WO 89/01940 filed September 1 1988 which are incorporated here fully by reference.

A wide choice of promoters is available for use in yeast cell expression systems and include by way on non limiting examples 3-phosphoglycerate kinase and one is directed to Hitzman et al J Biol Chem 255: 2073 (1980): also enolase, glyceraldehyde-3-phosphate dehydroginase, hexokinase, pyruvate decarboxylase, phosphofructokinase, gluocse-6-phosphate isomerase, 3-phosphoglycerate mutase, pyruvate kinase, triose phosphate isomerase, phospho glucose isomerase ad glucokinaise being glycolytic enzymes and one is directed to Hess et at J Adv Enzyme Reg 7; 149 (1968) and to Holland Biochemistry 17: 4900; 91978.

Other promoters suitable for yeast expression systems include the promoter regions for alcohol deyhydrognase 2, 100 cytocrome C, acid phosphatase, also mettallothioneins. glycoraldehyde-3-phosphate dehydrogenase and others one is directed to Hitzman R et al European patent publication No 73, 657A.

Where insect cells such as Lepidoptera cells are the chosen expression host a suitable vector would be Baculovirus. Such a system would contain the target peptide encoding sequence linked to a baculovirus promoter within a shuttle vector with sufficient baculovirus DNA flanking the target peptide encoding sequence to permit recombination.

One is directed to Summers et al TAES Bull (Texas Agricultural Experiemental Station Bulletin) NR 1555 May 1987.

One is also directed to SmithKlein (WO/US/89/05550).

Insect larvae can also be used to produce transformed insect cells, particularly Heliothis virescens caterpillars and one is direced to PCT/WO/88/02030 Miller at al.

Where plant cells are the chosen host expression cells the cowpea plant provides a suitable expression system. One is directed to the system developed by the Agriculture Genetics Company of Cambridge UK employing techniques involving the use of cowpea mosaic virus (CPMV).

Where mammalian cells are the chosen hosts for expression, these cells may be grown invitro in tissue culture or suitable bioreactors or in vivo in animals.

Vectors useful for mammalian cells host systems involve the use of DNA derived from animal viruses such as SV40 virus; retroviruses such as RSV, MMTV, MOMLV, baculovirus, Vaccinovirus, Adenovirus, polyoma or bovine papilloma virus.

Promoters suitable for mammalian cells systems may be chosen from the many available. One is directed to Friers et al Nature 273; 113 (1987) and Greenway P J et al Gene 18; 353-360 (1982) and Okayamah Mol Cell Biol 3; 280; 1; 1983 by way of example.

Additionally suitable enhancers may be chosen from the many available. One is directed to Laimins L et al PNAS 78; 993 (1981) and Lusky M L et al Mol Cell Bio 3; 1108 (1983) and Banerji J L et al Cell 33; 729 (1983) and Osbourne T F et al Mol Cell Bio 4; 1293 (1984).

For a description of some available selection techniques one is directed to Southern et al J Molec Appl Genet 1; 327 (1982) and to Mulligan et al Science 209; 1422 (1980) and to Sugden et al Mol Cell Biol 5; 410-413 (1985).

Some techniques useful for the introduction of the expression vector in to the host cell involve protoplast fusion, calcium phosphate precipitation, electroporation and other techniques as mentioned herein before.

The expression of the fusion peptide or fragment by the host may be confirmed by using an anti CD4 antibody such as a complimentary probe. One is directed to Dalgleish et al Nature 312; 763-766 (1984). Klatzman et al Immunol Today 7; 291-297 1986; McDougal et al J Immunol 153 135; 3151-3162 1985 and McDougal et al J Immunol 137 2937-2944 (1986) and to verification experiments mentioned herein after.

Mammalian host cells suitable for the expression of fragments of, or in some cases the entire fusion peptides may now be chosen from the large number now available such as VERO or CHO-K1, the hybridoma SP2\0-AG14 ot the myloma cell line P3x63Sgh or their P3xSgh derivatives also J558L, cells.

Other suitable eukaryotic host cells include COS cells; human embryonic kidney cells; mouse plasmacytoma cells; mouse sertoli cells; baby hamster kidney cells 9BHK cells); Chinese hamster ovary cells - DKFR (CHO); monkey kidney cells; African green monkey cells; human cervical carcinoma cells (HELA cells); human liver cells, and mouse mammary tumour cells by way of non limiting examples.

It will be appreciated that the chosen host cells will preferably express the minimum levels proteases within their cytoplasm. It will also be appreciated that amino acid sequence variations of the peptide sequences involved may be insertions, substitutional or deletional variations involving single amino acid residues or peptide fragments: combinations of or multiples of such variations, or strain or species variation.

The purpose of such variations may be to increase the affinity of the components, to improve stability, to reduce the cost of preparation or to increase the half life, or to lesson the severity of side effects such as atopic reactions. The final form of the molecular machines of the present disclosure may involve any combination of substitutions, delations or insertion of amino acid sequences, provided the binding ability of the epitopes or peptide sequences are retained. It will be appreciated that also included in the present disclosure are glycosylation variations ie variants completely unglycolated, variants having glycosylated amino acids other than those glycosylated in the natural peptides or variants having a greater number of amino acid residues glycosylated or fewer glycosylated residues or residues glycosylated by oligosaccharides other than those oligosaccharides usually associated with the said sequences.

Following their expression by the host cell, the peptides are recovered and purified by means known to the ordinary skilled artisan.

Such methods may include acid extraction, ethanol precipitation using amonium sulphate, anion or cation exchange chromatography, phosphocellulose chromatography, amino affinity chromatography, hydroxyappetite chromatography and reverse phase chromatography.

Generally purification and processing involves four stages:
1) extraction of peptides from host cells;
2) initial purification;
3) final purification:
4) product polishing.

Extraction may be accomplished using sonication techniques; or solid shear techniques - on a small scale.

Lysozyme based techniques are expensive. More usually in the case of bacterial hosts homogenisers or liquid shear techniques are employed.

Other useful techniques involve osmotic shock, freezing and thawing or alkali homogenisation.

In the case where a product is expressed as an inclusion body cell paste may be solublised by solvents such as 8M - guanidinium.

Centrifugation provides the removal cellular debris, and continuous flow centrifuges are preferred for large scale operations. As an alternative to centrafugation cross flow filtration using flat or tubular membranes and high shear forces may provide a useful alternative to centrifugation.

Initial purification involves mainly the removal of excess water and product concentration.

Precipitation using amonium sulphate, organic solvents, polyethylene glycol or other polymers can be used to acomplish this step: with the addition of some variety of chromatography usually absorptive chromotagraphy techniques employing ion exchange, hydrophobic or bioaffinity interactions; followed by washing and desorption; chromatography is not restricted to columns by may take place in membranes or even in spirally wound cartridges.

In cases where peptide fragments have been expressed as inclusion bodies an additional step of 'refolding' is required. Because of low yields after refolding inclusion body production is often uneconomical. However two approaches are practised to refold peptides into the natural or desired three dimensional state: the empirical approach and the rationalist approach.

In the empirical approach multiple solvents are applied and an optimum strategy is determined using phase diagrams as disclosed by Ahmed and Biglow 1979 J Mol Biol 131; 6097-6017

The rationalist approach seeks to produce conditions favouring the native state while at the same time keeping intermediates in solution.

A problem may arise in connection with disulphide bridges which may form in non-native configurations. A way around this problem is to oxidise disulphides under denaturing conditions, using gel filtration remove covalent aggregates and thereafter dilute the product in a non denaturing buffer. The peptide which collapses into an amorphous tangle often rearranges itself into the native form.

Highly resolving chromatography is the preferred technique for final purification. For large scale applications columns are preferred and techniques such as gel filtration, ion exchange, hydrophobic interaction or affinity chromatography may be used alone or in combination as dictated by economies of scale.

Gel filtration is best suited to small batch volumes and suffers from the disadvantage of slow speed.

Ion exchange chromtography techniques are very useful in early purification stages and can deal with large volumes at great speed, producing yields of high resolution.

Hydrophobic interaction chromatography provides both high resolution and high speed even at large batch volumes.

Bio affinity chromatography produces the highest resolution, at high speed, but batch size may require curtailment. This technique is an ideal late stage technique.

The increasing availability of monoclonal antibodies at lower prices has led to greater use of bio affinity chromatographic techniques.

A wide choice of chromatographic matrices is now available on the market suitable for large scale use.

Particle size is decreasing from the 90 um of traditional gels to approximately 40 um for newer gels such as Sepharose HR (R); Sephacryl HR (R); Superdex (R) (Pharmcia - LKB) or Fractogel (R) (Toso-Haas).

Other polymeric particles are Superose (R) (Pharmacia - LKB) or the TSK-PW (R) varieties manufactured by (Toso Haas, Philadelphia USA), providing very low particle size.

It will be appreciated that the process of chromatography involves the choice and development of a strategy continaing one or more steps ie high resolution by single step or a multistep procedure the final choice to be determined by:
1) the chosen peptide production process which determines the form of the starting materials to be purified
2) cost

Usually the first chromatography column will involve large diameter packings circa 100 um which are often chosen so that they can by resanitised by sodium hydroxide to reduce costs. Low resolution steps to be followed by higher reoslution steps until the desired product purity is obtained.

A typical strategy might involve:
- **Step One**: Hydrophobic interaction chromatography at low ionic strength. Purpose to absorb proteinases. Target peptides not absorbed and collected. Alternatively use proteinase inhibitors.
- **Step Two**: Rerun through hlc column adding salts to bind the target peptide to the hlc column. The objective is volume reduction. Alternatively employ ultra filtration as described. Use a step gradient to elute the target peptide.
- **Stage Three**: A step gradient to a low ionic stength buffer will remove remaining salts. Alternatively employ polyethyleneimine precipitation, centrifugation and diafiltration.

In the case of therapeutic peptides such as the fusion peptides of this disclosure where administration to a human is considered then the step of produt polishing is vital.

Product polishing involves the removal of polymers of the product and other pyrogens.

Techniques for product polishing involve additional gel filtration with or without a buffer exchange step: treatment with alyhdrogel (aluminim hydroxide) or treatment with specific lecithins or anion exchanges.

Following their extraction and purification the hybrid peptides may be combined with carriers, binders, preservatives, antioxidants, antimcrobial agents and the like to formulate a pharmaceutical composition; and one is directed to the British and American Pharmacopoea incorporated herein by reference. It is anticipated that intravenous administration of injectable compositions will be a principle route of administration of the hybrid peptides. However other compositions for administration include microphores, lyosomes or sustained release formulations, replantable or microcapsular sustained release matrices; intra muscular administration may also be used.

The hybrid peptides may also be components of capsules, tablets, creams, gels, lotions, drops, patches or may be administered where appropriate by any other of the known means of administration of a drug to a human where appropriate.

Hydro phobic solvents may be used for intravenous or parentral administration in some circumstances. Examples of such solvents are propylene glycol, polythylene glycol, vegetable oils, organic esters.

Aqueous carriers which are preferred are by way of example water for injection, emulsions or suspensions, saline or buffers, alcoholic aqueous solutions, sodium chloride solutions, ringers lactate or dextrose.

The manufacturing process only finishes with the secure packaging of the peptides in a suitable vacuum or atmosphere in suitable containers usually vials of glass or polymers with suitable stoppers. The said glass or polymer vials are then housed in a sturdy housing of cardboard or polymer after appropriate sealing. The cardboard housing will ideally be further wrapped in a clear polymer such as cellulose and provided with a seal to disclose tampering. All relevant information such as product name, date of manufacture, batch number and date of expriy and the like should be clearly visible and printed on the vials and housing as required by law and good manufacturing practice. The products should also be accompanied by leaflets, indicating material facts relevant to their use such as dosage, side effects, contra indications and the like it will be appreciated that the manufacturing process should take place in an appropriately designed facility, the floors, walls and ceilings of which should be finished to a high standard. Product flow must be arranged to reduce risk of mix up. Water systems employed should meet the US Pharmacopia specifications for pure water and be of injection quality in final purification stages.

Compressed air and nitrogen must be filtered through 0.2 u inch filters and be free of oil.

Final purification stages should ideally take place under laminate flow hoods or equivalent in suitably clean rooms.

In short the manufacture of the peptides must take place in conditions meeting GMP standards.

Validation and quality control are ongoing necessary steps to ensure public safety and product quality.

The disclosure will be illustrated by means of exemplary embodiments which are non limiting.

In the exemplary embodiments that follow a number of assumptions are made which will be appreciated by the reader, these assumptions to being common knowledge to persons skilled in the art.
1) It is generally considered easier by all arts from DNA cloning to woodworking to delete ie shorten an object than to add to it. Therefore peptide sequences as useful formulae longer than necessary will be provided by way of illustration: truncation or deletions to be carried out as desired.
2) Any substitutions and any modifications of the wild type CD4 or indeed the malaria erythrocyte binding molecules will fall within the scope and the spirit of the invention. Avi Ashkanazi et al Proceedings national Acadamey of Science USA Vol 87, p7150-7154 September 1990 reports substitution experiements where alanine residues were substituted for naturally occurring amino acid residues within the CD4 molecule. The results demonstrated that single alanine substitution made little difference in most cases to the ability of CD4 segments to bind to the HIV GP120. In some cases either no binding whatsoever was found or greatly improved binding was found more particularly the substitutions G4OA and D63A; of special interest are the double substitution G4OA and D63A which exhibited a 2.3 fold increase in affinity and the triple mutant Q40A D63A Q89L which exhibited a four fold affinity increase.
3) It is common knowledge that species variation or strain variations of peptides exist in nature. Accordingly it will be appreciated tha peptide segments derived from proteins of any strain of the malaria organism, filling the same role fall within the scope and spirit of the invention if used for the same purpose and in the 'same way' as the fusion peptides of the invention.

### EXEMPLARY EMBODIMENT NUMBER 1

Exemplary embodiment number 1 is directed to a hybrid protein which comprises a peptide sequence derived from the CD4 molecule or fragment thereof which possesses the capability of binding to HIV GP120, fused to a peptide sequence or fragment thereof derived from the merozoite glycophorin binding peptide GBP130.

The peptide sequence of the CD4 molecule providing derived fragments useful for the preparation for the first component was disclosed by Paul Jay Maddon et al in Cell, Vol 42, 93-104, August 1985. See Figure 6 page 97: incorporated fully herin by reference.

The second component peptide of the hybrid peptide of the present embodiment may be derived from the peptide sequence of GBP130 glycophorin binding peptide 130 as disclosed by Jarema Kochan et al in Cell Vol 44, 689-696 March 14 1986. See figure 2 page 691 and incorporated fully herein by reference.

In this example the peptide sequence consisting of 1-371 of the CD4 molecule is fused at the C terminal end to the N terminal end of a peptide sequence derived from the GBP130 molecule amino acid residues 201-774 of the GBP molecule or a fragment thereof. 
+1 371
Gln.............................. Val.................................. Ala
201 774
NH2-------------- CD4-------------|-------------------GBP130-----------------COOH
CN
 In this example the preferred fusion is by peptide bonds, it will also be appreciated that the site of fusion is illustrative but not limiting. Fusion may take place at other amino acid residues of both the above peptide components. The example will be further described by a useful formula of 
NH2-CD4--------------GBP130-COOH
1-371                    207-774
 to avoid any possible confusion

Therefore the formula of 
NH2-CD4-GBP130 COOH
1-371 201-774
 in would aspect would be, 
+1 10 20
N Terminal gln gly asn lys val val leu gly lys lys gly asp thr val glu leu thr cys thr ala ser
gln lys lys ser ile gln phe his trp lys asn ser asn gln ile lys ile leu gly asn gln gly ser phe
leu thr lys gly pro ser lys leu asn asp arg ala asp ser arg arg ser leu trp asp gln gly asn
phe pro leu ile ile lys asn leu lys ile glu asp ser asp thr tyr ile cys glu val glu asp gln lys
glu glu val gln leu leu val phe gly leu thr ala asn ser asp thr his leu leu gln gly gln ser leu
thr leu thr leu glu ser pro pro gly ser ser pro ser val gln cys arg ser pro arg gly lys asn
ile gln gly gly lys thr leu ser val ser gln leu glu leu gln asp ser gly thr trp thr cys thr val
leu gln asn gln lys lys val glu phe lys ile asp ile val val leu ala phe gln lys ala ser ser ile
val tyr lys lys glu gly glu gln val glu phe ser phe pro leu ala phe thr val glu lys leu thr gly
ser gly glu leu trp trp gln ala glu arg ala ser ser ser lys ser trp ile thr phe asp leu lys asn
lys glu val ser val lys arg val thr gln asp pro lys leu gln met gly lys lys leu pro leu his leu
thr leu pro gln ala leu pro gln tyr ala gly ser gly asn leu thr leu ala leu glu ala lys thr gly
 
lys leu his gln glu val asn leu val val met arg ala thr gln leu gln lys asn leu thr cys glu

val trp gly pro thr ser pro lys leu met leu ser leu lys leu glu asn lys glu ala lys val ser lys

arg glu lys ala val trp val leu asn pro glu ala gly met trp gln cys leu leu ser asp ser gly

gln val leu leu glu ser asn ile lys val leu pro thr trp ser thr pro val ser gln lys pro ser thr

ser thr arg ser asn asn glu val lys ile arg ala ala ser asn gln glu thr leu thr ser ala asp pro

glu gly gln ile met arg glu tyr ala ala asp pro glu tyr arg lys his leu glu ile phe tyr lys ile

leu thr asn thr asp pro asn asp glu val glu arg arg asn ala asp asn lys glu asp leu thr ser

ala asp pro glu gly gln ile met arg glu tyr ala ser asp pro glu tyr arg lys his leu glu ile phe

tyr lys ile leu thr asn thr asp pro asn asp asp val glu arg arg asn ala asp asn lys glu asp

leu thr ser ala asp pro glu gly gln ile met arg glu tyr ala ala asp pro glu tyr arg lys his leu

glu val phe his lys ile leu thr asn thr asp pro asn asp glu val glu arg arg asn ala asp asn

lys glu asp leu thr ser ala asp pro glu gly gln ile met arg glu tyr ala ala asp pro glu tyr

arg lys his leu glu ile phe his lys ile leu thr asn thr asp pro asn asp glu val glu arg arg

asn ala asp asn lys glu asp leu thr ser ala asp pro glu gly gln ile met arg glu tyr ala ala

asp pro glu tyr arg lys his leu glu val phe his lys ile leu thr asn thr asp pro asn asp glu

val glu arg arg asn ala asp asn lys glu leu thr ser ser asp pro glu gly gln ile met arg glu

tyr ala ala asp pro glu tyr arg lys his leu glu ile phe his lys ile leu thr asn thr asp pro asn

asp glu val glu arg arg asn ala asp asn lys glu asp leu thr ser ala asp pro glu gly gln ile
 
met arg glu tyr ala ala asp pro glu tyr arg lys his leu glu ile phe tyr lys ile leu thr asn thr

asp pro asn asp glu val glu arg arg asn ala asp asn lys glu asp leu thr ser ala asp pro glu

gly gln ile met arg glu tyr ala ala asp pro glu his leu glu ile phe his lys ile leu thr asn thr

asp pro asn asp glu val glu arg arg asn ala asp asn lys glu glu leu thr ser ser asp pro glu

gly gln ile met arg glu tyr ala ala asp pro glu tyr arg lys his leu glu ile phe tyr lys ile leu

thr asn thr asp pro asn asp glu val glu arg arg asn ala asp asn lys glu asp leu thr ser ala

asp pro glu gly  gln ile met arg glu tyr ala ser asp pro glu tyr arg lys his leu glu ile phe

tyr lys ile leu thr asn thr asp pro asn asp asp val glu arg arg asn ala asp asn lys glu asp

leu thr ser ala asp pro gly glu gln ile met arg glu tyr ala ala asp pro glu tyr arg lys his leu

glu ile phe his lys ile leu thr asn thr asp pro pro asn asp glu val glu arg gl:n asn ala asp

asn asn glu ala - C TERMINAL.
 which formula
describes the embodiment but is intended to be non limiting.

This exemplary embodiment is also directed to a protein gene which comprises a DNA sequence which codes for the CD4 molecule or fragment thereof which binds HIV GP120 fused to a DNA sequence which encodes the glyphorin 130 or fragment thereof which possesses the capability of binding to red cells.

DNA sequences suitable for forming the cloned DNA of the fusion peptide may be found in the disclosure of Paul Jay Maddon Cell, Vol 42 93-104, August 1985 see Figure 6 page 97. Some or all of the DNA sequence coding for the CD4 molecule as disclosed may be fused to the DNA sequence conditioning for the DNA glyphorin binding peptide. It may be preferable to include the leader sequence of the CD4 molecule. it may also be preferable to delete the transmembrane and cytoplasmic domains. A DNA sequence encoding the glycophorin peptide 130 and suitable for the fabrication for the cloned genes useful for the preparation of the hybrid peptides of this disclosure may be found in Jarema Kochan et al Cell Vol 4 689-696 March 14 1986 Figure 2 page 691. Suitable sites of fusion might be in the region of site 733 ie Leu AAA or in the vicinity of site 736 ie val, GTT or in the vicinity of Site 739 ie Ser TCT in the case of GBP130; and in the region of site 1257 for the CD4 derived sequence.

-17 +371 +202 +774
met.............................. val ser.................................. ala
76 1257 737 2455
ATG............................ GTG TCT..................................GCA

CD4 GBP130

By way of further illustration the CD4 DNA sequence is restricted at the BspM1 site position 514 and fused to some or all of the DNA sequence coding for GBP130 as disclosed above or restricted at other convenient sites

¦------- CD4----------¦--------------GBP130-------¦
BSPM1
514

The DNA sequence for the CD4 component as also obtained by restricting the DNA sequence encoding for the CD4 peptide component as previously disclosed at the Pvu11 site, position 691 and joined to the cloned DNA encoding the glycophorin binding peptide 130 fragment as previously described or smaller fragments thereof, restricted as desired.

¦------- CD4-----------¦-----------------GBP130------- ¦
Pvu11
629

Fusion will be carried out by suitable ligases and gene fragments, even those gene fragments encoding additional amino acids or sequences of amino acids may be inserted to enable the ligation provided the inserted segments do not interfere with the functionality of either the CD4 derived peptide or the GBP130 derived peptide segment.

It is also envisaged that the hybrid peptide will have 2 components: the first being a CD4 derived peptide sequence comprising amino acids 1-177 ie + 1 gln - 177 val fused to a glyophorin binding protein (GBP) 130 derived peptide sequence such as Leu 226-Ala 774 or Thr 227 Ala 274, fusion to take place at Leu 226 or Thr 227 by way of example fusion to consist of a peptide bond formed between CD4 amino acid 177 and the glycophrin binding peptide 130 amino acid 226 or 227 fused at the C terminal end of the CD4 derived peptide and the N terminal end of the GBP130 peptide sequence. The said sequences and the enumeration of the said sequences to be found in the sources mentioned herein before.

This variation of the embodiment contains only the tandem repeat sequence of the GBP-130 molecule.

Gln...............Val Thr.......................... Ala
+1 177 227 774
|------- CD4---------|-----------GBP130-----------|
C

In this exemplary embodiment it is also envisaged that a peptide sequence comprising the first 117 amino acids of the CD4 molecule or fragment thereof are fused C terminally to a peptide sequence of the glycophorin binding peptide GBP130. A suitable site of fusion would be Ala 370 N terminally to provide an amino acid lead sequence to be followed by the tandem repeat sequence beginning with Leu 376: the sequence terminating at Asp 425. It is appreciated that smaller sections of such a tandem sequence could be used as can smaller sections of the CD4 peptide sequence. The example is intended to be illustrative rather than limiting.

1                    177 370..376                    425
Gln--------------------    Val-Ala----Leu-----------------------    Asp

N---------------- CD4---------¦-----------    GBP-130----- C
                                        single tandem repeat

In all the preceeding variations of exemplary embodiment number 1 the CD4 component peptide may be fused at the N terminal end to the C terminal end of the GBP130 derived peptide sequence. It was shown by P Yeh et al PNAS March 1992 P1904-1908 that functionality is not lost when CD4 is fused N terminally.

### EXEMPLARY EMBODIMENT NUMBER 2

This exemplary embodiment is directed to a hydrbid peptide characterised by the fusion of some or all of the amino acid peptide sequence, amino acid residues 1-371 of the CD4 molecule fused at the C terminal end to a peptide sequence derived from the glycophorin binding peptide homologue molecule. Amino acid resides 70-427 or segments thereof of the glyophorin binding peptide homolgue(s). The amino acid sequence of the CD4 molecule has been disclosed by Paul Jay Maddon, the reference is given in Exemplary Embodiment No. 1. The glycophorin binding peptide homologue amino acid sequence is disclosed by Dagmar Nolte et al in Molecular and Biochem Parasitology 49, (1991) page 253-264 figure 2 page 257, incorporated fully herein by reference.

gln                    val-ser                    ser

N¦——CD₄ ——————————¦——GBPH ————¦C

+1                    371 7 0                    427

It may avoid confusion to describe the exemplary embodiment in one aspect by a useful formula which is intended to be non limiting.

Therefore the formula of 
NH2 CD4-GBPH-COOH
1-371 70-427
 in one aspect is:
N Terminal Q G N K V V L G K K G D T V E L T C T A S Q K K S I Q F H W K N S N
Q I K I L G N Q G S F L T K G P S K L N D R A D S R R S L W D Q G N F P L I I K N L
K I E D S D T Y I C E V E D Q K E E V Q L L V F G L T A N S D T H L L Q G Q S L T
L T L E S P P G S S P S V Q C R S P R G K N I Q G G K T L S V S Q L E L Q D S G T
W T C T V L Q N Q K K V E F K I D I V V L A F Q K A S S I V Y K K E G E Q V E F S
F P L A F T V E K L T G S G E L W W Q A E R A S S S K S W I T F D L K N K E V S
V K R V T Q D P K L Q M G K K L P L H L T L P Q A L P Q Y A G S G N L T L A L E A
K T G K L H Q E V N L V V M R A T Q L Q K N L T C E V W G P T S P K L M L S L K
L E N K E A K V S K R E K A V W V L N P E A G M W Q C L L S D S G Q V L L E S N
I K V L P T W S T P V S Q Y K Q A A D Y S F R E S R V L A E G K S T S K K N A K
T A L R K T K Q T T L T S A D P E G Q I M K A W A A D P E Y R K H L N V L Y Q I
L N N T D P N D E L E T S A D P E G Q I M K A Y A A D P E Y R K H L N V L Y Q I L
N N T D P N D E V E S S A D P E G Q I M K A Y A A D P E Y R K H V N V L Y Q I L
N N T D P N D E L E T S A D P E G Q I M K A Y A A D P E Y R K H V N V L Y Q I L N
H T D S S E V E T S A D P E G Q I M K A Y A A D P E Y R K H V N V L Y Q I L N H
T D S S E V E T S A D P E G Q I M K A Y A A D P E Y R K H V N V L Y Q I L N N T D
P N D E L E T S A D P E G Q I M K A Y A A D P E Y R K H V N V L Y Q I L N N T D P
N D E L E T S A D P E G Q I M K A Y A A D P E Y R K H V N V L Y Q I L N N T D P N
D E S S - C Terminal; which formula is but one possible variation of the many apparent to the skilled artisan.

This example is also directed to a fusion peptide which is characterised by the fusion of CD4 peptide sequence to a glycophorin binding peptide homologue sequence. The CD4 derived sequence comprises some or all of the amino acids from number 1-177 of the CD4 molecule. The sequence numbering and source of CD4 peptide sequence has been referenced herein before, see exemplary embodiment No. 1.

The above CD4 peptide sequence to be fused C terminally to the N terminal of glycophorin binding homologue peptide sequence amino acid residues 70-427 or some or part thereof. The source of glycophorin binding peptide homologue being the sequence disclosed by Dagmar Nolte (full reference given above).

gln                    val - ser                    ser
N¦---------CD4-----------------¦-----------------------GBPH---------------¦C
+1                     177  70                      427

This example is also directed to a hybrid peptide formed by the fusion of an amino acid peptide sequence derived from the CD4 molecule amino acid residues 1-371 or fragment thereof fused C terminally to a peptide sequence derived from the glycophorin binding peptide homologue fused N terminally at amino acid 109; the sequence comprising amino acids 109-427 some or part thereof. The sequencing of CD4 and glycophorin peptide binding homologue as already referenced.

gln val-leu ser

N CD4 GBPH C

+1 371 109 427

This example is also directed to a hybrid peptide which comprises the fusion of an amino sequence derived from the CD4 molecule the sequence as previously disclosed and consists of amino acid residues 1-177 or fragments thereof fused C terminally to a tandem repeat sequence(s) derived from the glycophorin peptide binding homologue molecule as disclosed herein before. Such a sequence may consist of amino acid residues 230-268. It will be appreciated that an even shorter fragment could be used. It will be also appreciated that one or more of the tandem repeat sequences could be included. It will be further appreciated that the tandem repeat sequence could be shortened to an even smaller fragment containing only the RBC binding epitopes: and that this smaller fragment could be repeated.

It will be still further appreciated that related glycophorin binding peptide homologue molecules whose sequences are known but not as yet disclosed in the public domain could provide peptide sequences - tandem repeat units - fulfilling the functions of GBP130 or the GBPH sequences as disclosed above "in the same way" without departing from the scope and spirit of the invention.

gln---------------------- val-thr---------------------------- glu

N¦-------------CD4 ........¦-------------GBPH-------.......¦C

1 177 230 single tandem 268

repeat

This example is further directed to a fused gene coding for a hybrid peptide comprising a amino acid sequence derived from the CD4 molecule joined to an amino acid sequence derived from the glycophorin binding peptide homologue molecule. A typical fused gene might consist of;

ATG.. CAG............CCC.................ACT..........GAA
76 144 514 1369 1488
-------CD4-------------X-----------GBPH-----------
Bspm1 single tandem
repeat

Where X is any or no intervening sequence, X being inspecified any non limiting.

Ligation to take place using ligases and additional linker segments by conventional means, known to the skilled artisan.

This example is further directed to a fusion gene consisting of a DNA sequence encoding a peptide derived from the CD4 molecule truncated at the Pvull site 691 fused to a single tandem repeat encoding DNA sequence derived from glycophorin binding peptide homologue gene as disclosed by Nolte as referenced herein before or part thereof.

ATG....CAG...........ACC.........ATC..........GAA
76 144 629 1369 1488
¦--------¦------- CD4---------¦-----X---------¦----GBPH---------¦
Pvu11 single tandem
repeat

Where X is any intervening sequence X being inspecified and non limiting. Suitable linkers and joining segments to be provided as desired.

All the preceding exemplary embodiments may be fused C terminally of CD4 segment to the N terminal of the GBPH component.

### EXEMPLARY EMBODIMENT NUMBER 3

This example is directed to hybrid peptide formed by the fusion of a peptide sequence derived from the CD4 molecule or fragment thereof: the amino acid resides 1-371 fused at their C terminal end to a peptide sequence derived from the EBA 175 antigen otherwise known as the erythrocyte binding antigen 175; amino acid residues 20-1435 all or part thereof. In this example some or all ie fragments of the component peptide sequences may be used to fabricate the hybrid peptide. The sequence of amino acids and DNA sequence of the gene encoding EBA 175 was disclosed by B Kim Lee Sim et al in the Journal Cell Biology Vol 111, 1990 p1877-1884, figure 2, p1880: incorporated fully herein by reference. The peptide sequence for CD4 being already referenced herein before.

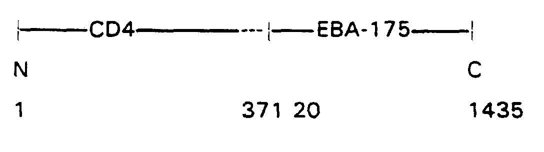
 CD4 EBA-175
N C
1 371 20 1435

To avoid any possible confusion it may be helpfyl to describe the embodiment by a useful formula which is intended as non limiting.

CD4 is truncated at 371 and EBA-175 truncated at 19 ie excluding the hydrophobic sequence. CD4 after Maddon. EBA-175 after Kim Lee Sim J Cell Biology Vol 111 1990, 1880

Therefore the molecular machines 
NH2 CD4-EBA 175-COOH
1-371 20-1435
 in one aspect would be:
N Terminal Q G N K V V L G K K G D T V E L T C T A S Q K K S I Q F H W K N S N
Q I K I L G N Q G S F L T K G P S K L N D R A D S R R S L W D Q G N F P L I I K N L
K I E D S D T Y I C E V E D Q K E E V Q L L V F G L T A N S D T H L L Q G Q S L T
L T L E S P P G S S P S V Q C R S P R G K N I Q G G K T L S V S Q L E L Q D S G T
W T C T V L Q N Q K K V E F K I D I V V L A F Q K A S S I V Y K K E G E Q V E F S
F P L A F T V E K L T G S G E L W W Q A E R A S S S K S W I T F D L K N K E V S
V K R V T Q D P K L Q M G K K L P L H L T L P Q A L P Q Y A G S G N L T L A L E A
K T G K L H Q E V N L V V M R A T Q L Q K N L T C E V W G P T S P K L M L S L K
L E N K E A K V S K R E K A V W V L N P E A G M W Q C L L S D S G Q V L L E S N
I K V L P T W S T P V K A R N E Y D I K E N E K F L D V Y K E K F N E L D K K K Y
G N V Q K T D K K I F T F I E N K L D I L N N S K F N K R W K S Y G T P D N I D K N
M S L I N K H N N E E M F N N N Y Q S F L S T S S L I K Q N K Y V P I N A V R V S
R I L S F L D S R I N N G R N T S S N N E V L S N C R E K R K G M K W D C K K K N
D R S N Y V C I P D R R I Q L C I V N L S I I K T Y T K E T M K D H F I E A S K K E S
Q L L L K K N D N K Y N S K F C N D L K N S F L D Y G H L A M G N D M D F G G Y
S T K A E N K I Q E V F K G A H G E I S E H K I K N F R K E W W N E F R E K L W E
A M L S E H K N N I N N C K N I P Q E E L Q I T Q W I K E W H G E F L L E R D N R
S K L P K S K C K N N T L Y E A C E K E C I D P C M K Y R D W I I R S K F E W H T
L S K E Y E T Q K V P K E N A E N Y L I K I S E N K N D A K V S L L L N N C D A E
Y S K Y C D C K H T T T L V K S V L N G N D N T I K E K R E H I D L D D F S K F G
C D K N S V D T N T K V W E C K N P Y I L S T K D V C V P P R R Q E L C L G N I D
R I Y D K N L L M I K E H I L A I A I Y E S R I L K R K Y K N K D D K E V C K I I N K
T F A D I R D I I G G T D Y W N D L S N R K L V G K I N T N S K Y V H R N K K N D
K L F R D E W W K V I K K D V W N V I S W V F K D K T V C K E D D I E N I P Q F F
R W F S E W G D D Y C Q D K T K M I E T L K V E C K E K P C E D D N C K S K C N
S Y K E W I S K K K E E Y N K Q A K Q Y Q E Y Q K G N N Y K M Y S E F K S I K P
E V Y L K K Y S E K C S N L N F E D E F K E E L H S D Y K N K C T M C P E V K D V
P I S I I R N N E Q T S Q E A V P E E N T E I A H R T E T P S I S E G P K G N E Q K E
R D D D S L S K I S V S P E N S R P E T D A K D T S N L L K L K G D V D I S M P K
A V I G S S P N D N I N V T E Q G D N I S G V N S K P L S D D V R P D K K E L E D
Q N S D E S E E T V V N H I S K S P S I N N G D D S G S G S A T V S E S S S N T G
L S I D D D R N G D T F V R T Q D T A N T E D V I R K E N A D K D E D E K G A D E
E R H S T S E S L S S P E E K M L T D N E G G N S L N H E E V K E H T S N S D N V
Q Q S G G I V N M N V E K E L K D T L E N P S S S L D E G K A H E E L S E P N L S
S D Q D M S N T P G P L D N T S E E T T E R I S N N E Y K V N E R E D E R T L T K
E Y E D I V L K S H M N R E S D D G E L Y D E N S D L S T V N D E S E D A E A K M
K G N D T S E M S H N S S Q H I E S D Q Q K N D M K T V G D L G T T H V Q N E
I S V P V T G E I D E K L R E S K E S K I H K A E E E R L S H T D I H K I N P E D R N
S N T L H L K D I R N E E N E R H L T N Q N I N I S Q E R D L Q K H G F H T M N N
L H G D G V S E R S Q I N H S H H G N R Q D R G G N S G N V L N M R S N N N N
F N N I P S R Y N L Y D K K L D L D L Y E N R N D S T T K E L I K K L A E I N K C E
N E I S V K Y C D H M I H E E I P L K T C T K E K T R N L C C A V S D Y C M S Y F
T Y D S E E Y Y N C T K R E F D D P S Y T C F R K E A F S S M I F K F L I T N K I Y
Y Y F Y T Y K T A K V T I K K I N F S L I F F F F F S F -
C Terminal, which is intended to be non limiting. viarations of which will be apparent to the skilled artisan.

This example is also directed to a fusion peptide comprising the fusion of a peptide sequence derived from CD4 consisting of the first 177 amino acid residues or fragment peptide sequence thereof: amino acid 1-177 fused at the C terminal end to the N terminal end of a peptide sequence derived from EBA 175 amino acid 1062-1103. This particular peptide sequence is also named EBA peptide 4 and has the ability to inhibit the binding of EBA 175 to red blood cells. Also included would be fused genes formed by the fusion of DNA sequences encoding the CD4 molecule fused to Sequences encoding the erythrocyte binding antigen 175 or its derived epeptide EBA peptide 4. The DNA sequences of the EBA peptide number 4 and EBA 175 molecules may be obtained from the Journal of Cell Biology Vol 111 1990, p 1880 in the article by J Kim Lee Sim et al in the same journal.

N¦----- -CD4 - - - - - - - - ----¦- - • •EBA peptide 4- - - - - -¦C
1                    177        1062                    1103

It is also envisaged that the CD4 component may be fused at the N terminal end to the C terminal end of any of the preceding examples.

### EXEMPLARY EMBODIMENTS USING P. VIVAX DUFFY

### EXEMPLARY EMBODIMENT NUMBER 4

In this embodiment the P Vivax Duffy Receptor molecule is joined to the CD4 receptor molecule both truncated at their transmembrane extrecellular domain regions.

¦-P Vivax Duffy·····|--¦ ¦--+···CD4 J1-4 V1-4--¦
Tm Tm
deleted Tm region

New molecule

H2N- P Vivax Duffy-C C —CD4 J1-4 V1-4---NH2
23->1051 371<-1
J
J segment or cross linker

Obviously further deletions and substitutions may be made to this molecule without departing from its intended function, the listing of all possible deletions and substitutions obvious to an artisan skilled in the art would be tedious and is excluded in the interests of brevity.

It is to be noted that the amino acid sequence of CD4 as presented in this embodiment is the same as for Maddon P N A Sci 1987 but in reverse order.

It is also to be noted that the CD4 peptide segment may be fused at the N terminal end to the C terminal of the P Vivax Duffy peptide, or at the C terminal end of the CD4 molecule to the N terminal of the P Vivax Duffy Receptor; by peptide bonds. This embodiment is also directed to a hybrid peptide comprosing the fusion or joining together of two amino acid sequences; one derived from the CD4 molecule and consisting some of a fragment of amino acid or peptide sequence derived from plasmodium Vivax Duffy Receptor molecule or fragment thereof: amino acid sequence 23-1051 joined at the C terminal ends which will require that the component sequences be expressed separately in separate hosts and fused subsequently. Many strategies are available for joining two peptide sequences together at both C terminal ends. One strategy envisages terminating both peptide sequences with 'cys' or incorporating cys near each C terminal end and fusion of the two sequences by means of sulphidryl bonds. Other strategies involve the use of joining sequences containing cys facilitating sulphidryl bonds linking the joining segment to the component peptide sequences. The genetic sequence and amino acid sequence of Plasmodium Vivax Duffy Receptor is disclosed by Xiangdang Fang et al in Molecular and Biochemical Parasitology 44, (1991) 125-132 one is directed to figure 1 of p 127.

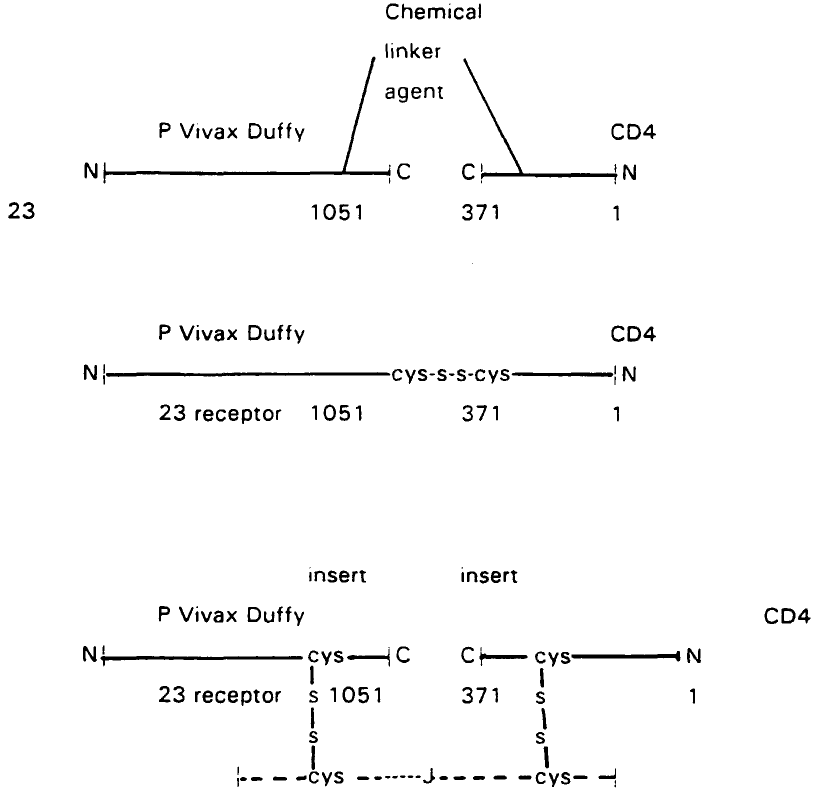
Chemical
linker
agent
P Vivax Duffy
CD4
NC CN
23
1051
371
1
P Vivax Duffy
CD4
Ncys-s-s-cysN
23 receptor
1051
371
1
insert
insert
P Vivax Duffy
CD4
NcysC CcysN
23 receptor
s 1051
371
s
1
s
s
cys
J
cys

It may be helpful to decribe the fusion peptides by a useful formula of a amino acids, the useful formula is intended to be non limiting.

Therefore a useful formula

for N —— P Vivax Duffy —— C C —— CD4 J1-4 VI-4 —————— N
8 23 -> 1051 371 <- 1
J

Where J is a joining segment or cross linker in one aspect would be:
K D D F S I T L I N Y H E G K K Y L I I L K R K L E K A N N R D V C N F F L H F S Q
V N N V L L E R T I E T L L E C K N E Y V K G E N G Y K L A K G H H C V E E D N L
E R W L Q G T N E R R S E E N I K Y K Y G V T E L K I K Y A Q M N G K R S S R I L
K E S I Y G A H N F G G N S Y M E G K D G G D K T G E E K D G E H K T D S K T D
N G K G A N N L V M L D Y E T S S N G Q P A G T L D N V L E F V T G H E G N S R
K N S S N G G N P Y D I D H K K T I S S A I I N H A F L Q N T V M K N C N Y K R K
R R E R D W D C N T K K D V C I P D R R Y Q L C M K E L T N L V N N T D T N F H
R D I T F R K L Y L K R K L I Y D A A V E G D L L L K L N N Y R Y N K D F C K D I R
W S L G D F G D I I M G T D M E G I G Y S K V V E N N L R S I F G T D E K A Q Q R
R K Q W W N E S K A Q I W T A M M Y S V K K R L K G N F I W I C K L N V A V N
I E P Q I Y R W I R E W G R D Y V S E L P T E V Q K L K E K C D G K I N Y T D K K
V C K V P P C Q N A C K S Y D Q W I T R K K N Q W D V L S N K F I S V K N A E K
V Q T A G I V T P Y D I L K Q E L D E F N E V A F E N E I N K R D G A Y I E L C V C
S V E E A K K N T Q E V V T N V D N A A K S Q A T N S N P I S Q P V D S S K A E
K V P G D S T H G N V N S G Q D S S T T G K A V T G D G Q N G N Q T P A E S D
V Q R S D I A E S V S A K N V D P Q K S V S K R S D D T A S V T G I A E A G K E
N L G A S N S R P S E S T V E A N S P G D D T V N S A S I P V V S G E N P L V T P
Y N G L R H S K D N S D S D G P A E S M A N P D S N S K G E T G K G Q D N D M
A K A T K D S S N S S D G T S S A T G D T T D A V D R E I N K G V P E D R D K T
V G S K D G G G E D N S A N K D A A T V V G E D R I R E N S A G G S T N D R S K
N D T E K N G A S T P D S K Q S E D A T A L S K T E S L E S T E S G D R T T N D T
T N S L E N K N G G K E K D L Q K H D F K S N D T P N E E P N S D Q T T D A E G
H D R D S I K N D K A E R R K H M N K D T F T K N T N S H H L N S N N N L S N G
K L D I K E Y K Y R D V K A T R E D I I L M S S V R K C N N N I S L E Y C N S V E D
K I S S N T C S R E K S K N L C C S I S D F C L N Y F D V Y S Y E Y L S C M K K E
F E D P S Y K C F T K G G F K 
-COOH    CROSS LINKER    COOH  V P T S E T P
terminal
OR
JOINING
SEQUENCE
insert cys in Duffy    insert cys in CD4
and insert cys in    and insert cys in
J sequence    J    J sequence
L V K I N S E L L V Q G S D S L L C Q W M G A E P N L V W V A K E R K S V K A E
K N E L K L S L M L K P S T P G W V E C T L N K Q L Q T A R M V V L N V E Q H L
K G T K A E L A L T N N G S G A Y Q P L A Q P L T L H L P L K K G M Q L K P D Q
T V R K V S V E K N K L D F T I W S K S S S A R E A Q W W L E G S G T L K E V T
F A L P F S F E V Q E G E K K Y V I S S A K Q F A L V V I D I K F E V K K Q N Q L V
T C T W T G S D Q L E L Q S V S L T K G G Q I N K G R P S R C Q V S P S S G P P
S E L T L T L S Q G Q L L H T D S N A T L G F V L L Q V E E K Q D E V E C I Y T D
S D E I K L N K I I L P F N G Q D W L S R R S D A R D N L K S P G K T L F S G Q N
G L I K I Q N S N K W H F Q I S K K Q S A T C L E V T D G K K G L V V K N G Q
NH₂ Terminal;
the numbering of this useful formula is after that of the referenced sourced peptides. It will be appreciated that the skilled artisan could provide countless variations on the above without departing from the scope and spirit of the disclosure.

### EXEMPLARY EMBODIMENT NUMBER 5

This example is directed to a hybrid peptide formed by the fusion of a peptide sequence derived from the P Knowlesi Duffy Receptor binding molecule as disclosed by Xiangdong Fang in Molecular and Biochemical Parasitology 44, (1991) 125-132 see figure 1 P 127. The choice of peptide fragment is non critical provided it contains erythrocyte binding ability and the method of joining to the CD4 component is also non critical, examples of typical methods of joining peptides are given in the preceding examples.

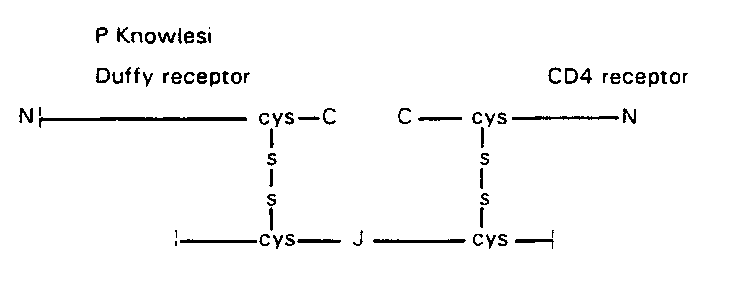
P Knowlesi
Duffy receptor
CD4 receptor
Ncys—C C—cys—N
s s
s s
cys—J—cys

The fusion may also be by peptide bonds N terminal of CD4 component to C terminal of P Knowlesi or N terminal of P Knowlesi component to C terminal of CD4 component

### EXEMPLARY EMBODIMENT NUMBER 6

This example is directed to hybrid peptide formed by the fusion of a peptide sequence derived from the expression of the PMMSA gene or precursor to the major merozoite surface antigen genes as disclosed by M Gregory Peterson in Molecular and Biochemical Parasitology, 27 (1988) P291-302. One is directed to p294 and p295 figure 3.

In this example an amino acid sequence derived from the expression of nucleic acid residues 177-5169 all or part fused at the C terminal end to the C terminal end of the CD4 derived peptide sequence such as formed from the expression of nucleic acid residues 114-1260 or fragment thereof.

The fusion at the C terminal end being accomplished by suitable joining sequences chemical ligands or sulphidryl bonds or other means.

It will also be envisaged that cys residues be added as required as described in exemplary embodiment number 14: the site of insertion is non critical providing binding function is maintained.

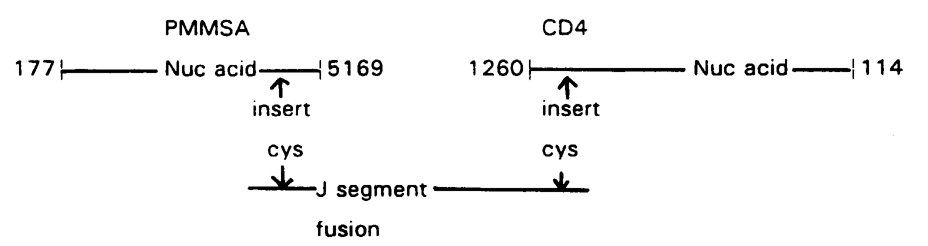
PMMSA                                        CD4
177 Nuc acid 5169        1260 Nuc acid 114
insert                                       insert
cys                                          cys
 J segment
fusion

Alternatively a fused gene joining the CD4 component to the PMMSA component N terminally to C terminally by peptide bonds may be utilised to provide alternative variations apparent to the skilled artisan.

### EXEMPLARY EMBODIMENT NUMBER 7

Exemplary embodiment number 7 contemplates joining sequences suitable for joining two peptide fragments at the C terminal ends. The joining sequences could be a straight peptide chain which will have cys amino acid residues added to form sulphidryl bonds with cys residues also inserted into the peptide sequences to be joined. Other methods of ligation are also envisaged. A suitable straight segment, but by no means the only suitable straight segment may be found in the joining chain amino acid resodues 95-112 inclusive of the CD4 molecule; the sequence disclosed by Paul Jay Maddon and already referenced herein before. 'Cys' residues may be inserted at any site for example between amino acid 97 and 98 and between 107 and 108 to name but two possible sites.

The CD4 molecule is part of the immunoglobulin superfamily.

The immunoglobulin superfamily provide many examples of straight and bent chains with cys already inserted for the purposes of such bonding.

One is free to choose from any of these peptide segments without departing from the scope and spirit of the invention as disclosed.

One is also free to use other straight or bent peptide sequences.

N H2 val.gln.leu.leu.val.phe.gly.leu.thr.ala.asn.asn.ser.asp.thr.his.leu.leu.gln. C Term
insert insert
cys cys

### EXEMPLARY EMBODIMENT NUMBER 8

Exemplary embodiment number 8 illustrates how a joining sequence J can be used to join an EBA175 fragment with a GBP130 fragment and both to be joined to a CD4 derived peptide or variations already listed. The J peptide sequence may be similar to that already provided or have additional cys residues added.

GBP130 EBA-175
774 cys 202 20 cys 1435
s s
s s
--J segment-cys cys cys
s
s
371 cys 1
CD4

It will be appreciated that branched chains such as the one illustrated or variations thereof, anyone of which functioning in the same way would be apparent to the skilled artisan and fall within the scope and spirit of the disclosure.

### EXEMPLARY EMBODIMENT NUMBER 9

This example envisages fusion peptides formed by the fusion of ZOOANOTIC MEROZOITE derived peptide fragments whose function in nature is to bind with non human red blood cells, fused to CD4 segments in the manner already suggested.

Useful sources of peptides are
Plasmodium Chabaudi
Plasmodium Yeolii
Plasmodium Berghei
Plasmodium Gallinacium
Plasmodium Cyanomogli

The molecular machine so produced would be

(ZOONOSIS)
Animal parasite
merozite derived | CD4
RBC binding peptide |

The amino acid sequences of such peptides are to be found in the Protein Data Bases together with restriction maps, in the public domain, available on payment; of a fee and incorporated fully herin by reference. Such novel fusion peptides find a use in HIV testing

### METHOD OF USE OF EXEMPLARY EMBODIMENT 9

A sample of human blood to be tested is mixed with a known quantity of animal blood treated with the molecular machines of example 9.

The sample is agitated and thereafter the animal RBC removed selectively by anti-animal RBC antibodies bound to argarose or polyacrylamide gels or the like. Fluorescent labelled anti HIV antibody is then employed demonstrate virus particles adherent to the animal red cell. Quantification may then take place by automated systems in the usual way.

### EXPERIMENTAL VERIFICATION

### Experiment 1 A: Immunological characterisation using antibodies directed against CD4

Purified samples of the fusion peptides in amounts ranging from 0.1 ug to 1 ug are used to coat the walls of ELISA plates and are thereafter incubated with preparations of OKT4A mAb (Ortho Diagnostics): the activity of the OKT4A are first altered by incubation with varying amounts of soluble CD4 preparations. A labelled anti-mouse serum is then used to assess the amount of bound mouse antibody.

### Experiment 1 B: Immunological Characterisation using antibodies directed against the malaria derived component

Purified samples of the fusion peptides in amounts ranging from 0.1 ug to 1 ug are used to coat the walls of ELISA plates and are thereafter incubated with monoclonal antibodies directed against the erythrocyte binding peptide component. Binding is revealed by goat anti mouse antibodies the said goat antibodies to be labelled by conventional means.

### Experiment 2: The binding of the disclosed molecular machines to red cell surfaces.

A buffered solution containing 100,000 human red blood cells is incubated with 20 ug of fusion peptides. The washed red blood cells are thereafter incubated with 1 x 10¹¹ M of OKT4A (Ortho Diagnostics). The RBC's are washed again with appropriate isotonic buffer and incubated with peroxidase labelled goat anti mouse IGG serum (BIOYSIS). Further washing and the addition of 3, 3', 5, 5' - tetramethylbenzidine permits assessment of binding by the measurement of absorbance at 600 nm.

### Experiment 3: The binding kinetics of RBC: and the disclosed fusion peptides

A known quantity of a purified sample of fusion peptide 0.1 ug to 1 ug in increments of x 2 are incubated with 100,000 RBC's in isotonic buffer solution. 125-I labelled OKT4A or otherwise labelled OKT4A are added at set times 1 min, 2 min, 4, 8, 16, 30 min and 1 hour; the red cells arecentrifuged through Percoll 2 minutes after the addition of 125-I labelled OKT4A. Accordingly both the optimum ratio of RBC to molecular machines can be calculated together with the binding time to saturation of each incremental dose of fusion peptides.

### Experiment 4: The binding of live virus to red blood cells treated with the disclosed fusion peptides

10 ml of whole blood drawn from HIV Elisa and WB negative blood donors is centrifuged after mixing with Alserer's solution and rediluted to haematocrits of 25%, 35%, 45%, 55% and 65%, 75% this corresponding to the range of haematocrits seen in gross anaemia with dilution, to packed cells. The whole blood samples are incubated with amounts of fusion peptides ranging from 0.1 ug in increasing increments of x 2 to 10 ug. Thereafter the samples are incubated after mixing with 1 ml of suspension of cultured HIV-1 Bru particles having viral dose levels ranging from 50 to 50,000 viral particles per ml. Centrifugation is carried out after 60 minutes. The supernatant and red blood cell are separately assayed for bound virus using techniques already described, or by conventional means.

### Experiment 5: In vitro demonstration of clinical utility

50 ml samples of blood are obtained from HIV positive and AIDS patients and divided into of 10 ml after appropriate buffering and anticoagulation. One 10 ml sample from each patient is assayed for viral titre levels by conventional means. The remaining 4 samples of 10 ml of blood from each patient are treated by fusion peptides of exemplary embodiments 1,2,3,4,5,6 and 8 in amounts ranging from 0.1 ug to 1 mg and thereafter assayed for viral titre levels by conventional means. Additional smaples are centrifuged and the packed cells rediluted and assayed for viral titre levels separate from the plasma also assayed.

## Claims

1. A hybrid fusion peptide for use in therapy formed by the fusion of two or more components where one component is a peptide sequence of substitutional or deletional or insertional or strain or species variant of a peptide sequence with affinity for red blood cells derived from a malaria parasite merozoite peptide especially segments of the glycophorin binding peptide 130 (GBP130), or the glycophorin binding peptide homologues (GBPH), or the erythrocyte binding antigen 175 (EBA175), or the plasmodium vivax Duffy receptor or the pre major merozoite surface antigen (PMMSA) or the plasmodium falciparum merozoite surface antigen (Pf200) fused to all or part of a virus binding peptide segment derived from a viral receptor or substitutional or deletional or insertional virus binding variant thereof.

2. A hybrid fusion peptide according to claim 1 wherein the viral receptor is any of
(a) CD4,
(b) an immunoglobulin Fab segment having affinity for blood borne viruses and
(c) a hepatitis B, C or D viral binding receptor or hepatitis virus binding antibody.

3. A fusion peptide according to claim 2 wherein the C terminal end of CD4 is joined to the N terminal end of the glycophorin binding peptide GBP130 the resulting fusion peptide being characterised as

NH2-CD4——GBP 130-COOH
1-371   201-774.

4. A fusion peptide according to claim 3 comprising all or part of CD4 amino-acid residues 1-371 fused to all or part of GBP130 amino acid residues 201-774, the GBP130 part being fused to the CD4 part either N or C terminally.

5. A fusion peptide according to claim 2 wherein the first 177 amino acid residues, all or part, of the CD4 molecule are joined at their C terminal end to the N terminal end of the peptide sequence derived from glycophorin binding peptide 130 amino acid residues 201-774. The resulting peptide sequence being

NH2-CD4—GBP 130-COOH
1-177 201-774.

6. A fusion peptide according to claim 2 wherein all or part of the peptide sequence comprising amino acids 1-177 of the CD4 molecule is fused at the C terminal end to the N terminal end of the peptide sequence all or part derived from amino acids 1-774 of the glycophorin binding peptide 130

NH2-CD4——GBP 130-COOH
1-177 1-774.

7. A fusion peptide according to claim 2 comprising the CD4 derived peptide sequence, all or part, of amino acid residues 1-177 of CD4 fused at its C terminal end to the N terminal end of glycophorin binding peptide homologue GBPH residues 70-427

NH2-CD4- GBPH-COOH
1-371   70-427.
177

8. A fusion peptide according to claim 2 comprising the CD4 derived peptide sequence 1-371 all or part fused at its C terminal end to the N terminal end of glycophorin binding peptide homologue GBPH residues 70-427 all or part

NH2-CD4- GBPH-COOH
1-371 70-427.

9. A fusion peptide according to claim 2 comprising the human CD4 derived peptide sequence amino acid residues 1-371 all or part fused at the C terminal end to the N terminal end of amino acid residues of glycophorin binding protein homologue GBPH sequence 109-427

NH2-CD4—GBPH-COOH
1-371    109-427.

10. A fusion peptide according to claim 2 formed from the fusion of peptide sequences derived from all or part of the CD4 molecule amino acid residues of 1-371 fused at their C terminal end to the N terminal end of EBA 175, Erythrocyte Binding Antigen 175, a malaria peptide, residues 20-1435 all or part, and especially that part containing amino acid residues 1062-1103 Of EBA175 also referred to as EBA peptide4; the general formula being:

NH2-CD4- EBA175-COOH
1-371 20-1435.

11. A fusion peptide according to any one of claims 2 to 10, where the CD4 component is fused at the N terminal end to the C terminal end of the malaria derived peptide component.

12. A fusion peptide according to claim 2 formed by the fusion of a peptide segment derived from the plasmodium vivax Duffy receptor molecules, all or part of the peptide segment amino acid residue 23-1051, joined at the C terminal end to the C terminal end of the peptide segments derived from the CD4 molecule all or part of peptide segments comprising amino acid residues 1-371 of CD4; the said fusion or joining to take place by joining amino acid segments at sites unspecified by disulphide bonds, by crosslinking agents of any type

NH2—P Vivax—IC CI————————CD4—NH2
Duffy
23-1051                    371-1

—— J————————.
unspecified joining segment

13. A fusion peptide according to claim 2 toned by the fusion of peptide segments derived from the Pf200 or PMMSA malaria parasite molecule all or part joined at the C terminal end to a CD4 derived peptide comprising amino acid residues 371-1; the said fusion or joining to take place by joining amino acid segments at sites unspecified by disulfide bonds, by crosslinking agents of any type

NH2-PMMSA——C C———CD4-NH2
peptide peptide
segment segment
coded by coded by
nucleic acid nucleic acid
175-5169 1260-114
—— J———
Joining arm.

14. A fusion peptide formed from a segment of the P Knowlesi Duffy receptor molecule joined at its C terminal ends to the C terminal end of CD4 peptide all or part comprising amino acid residues 371-1 of CD4; the said fusion or joining to take place by joining amino acid segments at sites unspecified by disulfide bonds, by crosslinking agents of any type

NH2—P Knowlesi—C C————————CD4—NH2
Duffy 371-1
———J————.

15. A fusion peptide according to any one of claims 12, 13 and 14 where the CD4 derived component is joined N terminally to the C terminal of the merozoite derived peptide fragment.

16. A fusion peptide according to any one of claims 12, 13 and 14 where the merozoite derived peptide fragment is joined N terminally to the C terminal of the CD4 peptide fragment.

17. A fusion peptide according to any one of claims 2 to 16 wherein there is interposed between the CD4 derived peptide and the merozoite derived peptide, a peptide fragment comprising all or part of the following sequence of amino acids:
S T K G P S V F P L A P S S K S T S G G T A A L G C L V K
D Y F P E P V T V S Y N S G A L T S G V H T F P A V L Q S
S G L Y S L S S V V T V P S S S L G T Q T Y I C N V N H K
P S N T L V D K K V E P K S C D K T H T C P P C P A P E L
L G G P S V F L F P P K P K D T L M I S R T P E V T C V V
V D V S H E D P E V K F N W Y V D G V E V H N A K T K P R
E Q Y N S T Y R V V S V L T V L H Q D W L N G K E Y K C K
V S N K A L P A P I E K T I S K A K G Q P R E P Q V Y T L
P P S R D E L Y K N Q V S L T C V K G F Y P S D I A V E W
E S N G Q P E N N Y K T T P P V L D S D G S F F L Y S K L
T V D K S R W Q Q G N V F S C S V M H E A L H N G Y T Q K
S L S L S P G K.

18. A protein gene encoding a fusion peptide according to any one of the preceding claims.

19. A fusion peptide according to any one of claims 1 to 17 for use
(a) in the treatment of HIV 1 or HIV 2 infection; or
(b) in preventing the transmission of HIV 1 or HIV 2; or
(c) in lowering viral titres of HIV 1 or HIV 2 infected persons; or
(d) in improving the usefulness of blood transfusions or their safety.

20. A fusion peptide according to any one of claims 1 to 17 in the form of eye drops.

21. A fusion peptide according to claim 19 in the form of a vaccine directed against HIV 1 or HIV 2 infection.

22. The use of a fusion peptide according to any one of claims 1 to 17 for the manufacture of a medicament
(a) for therapeutic use as a contraceptive agent in the form of either foam, cream, spray, jelly or delayed release agent incorporated into a contraceptive device; or
(b) for treating HIV 1 or HIV 2 infection.

23. Use according to claim 22 wherein the medicament is in the form of a vaccine directed against HIV 1 or HIV 2 infection.

24. Use of a fusion peptide according to any one of claims 1 to 17 for the manufacture of a medicament for the prevention or treatment of hepatitis B, C or D.

25. A testing kit for intended use for the detection of HIV infection or the quantification of viral titre levels and comprising a fusion peptide according to any one of claims 1 to 17.

## Patentansprüche

1. Ein hybrides Fusionspeptid für eine therapeutische Verwendung, das durch die Fusion von zwei oder mehreren Komponenten gebildet ist, wo eine Komponente eine Peptid-Sequenz einer substitutionalen oder deletionalen oder insertionalen oder Stamm- oder Species-Variante einer Peptid-Sequenz mit einer Affinität für rote Blutzellen ist und von einem Malaria-Parasiten Merozoiten-Peptid abstammt, besondere Segmente des Glycophorin-bindenden Peptide 130 (GBP130) oder der Glycophorin-bindenden Peptid-Homologen (GBPH) oder des Erythrocytenbindenden Antigens 175 (EBA175) oder des Plasmodium vivax Duffy Rezeptors oder des Vorläufers des Haupt-Merozoiten-Oberflächen-Antigens (PMMSA) oder des Plasmodium falciparum Merozoiten Oberflächen-Antigens (Pf200), und an das gesamte oder einen Teil eines Virus-bindenden Peptid-Segments, das von einem viralen Rezeptor oder einer substitutionalen oder deletionalen oder insertionalen Virusbindenden Variante davon abstammt, fusioniert ist.

2. Ein hybrides Fusionspeptid gemäß Anspruch 1, worin der virale Rezeptor ein beliebiger Rezeptor ist aus
(a) CD4,
(b) einem Immunoglobulin Fab Segment mit einer Affinität für im Blut beförderte Viren und
(c) einem Hepatitis B, C oder D viralen bindenden Rezeptor oder Hepatitis-Virus bindenden Antikörper.

3. Ein Fusionspeptid gemäß Anspruch 2, worin das C-terminale Ende von CD4 mit dem N-terminalen Ende des Glycophorin-bindenden Peptids GBP130 verbunden ist, wobei das resultierende Fusionspeptid gekennzeichnet ist durch

NH2-CD4—GBP 130-COOH
1-317    201-774.

4. Ein Fusionspeptid gemäß Anspruch 3, das alle oder einen Teil der CD4 Aminosäure-Reste 1-371, die an alle oder einen Teil der GBP130 Aminosäure-Reste 201-774 fusioniert sind, umfasst, wobei der GBP130 Teil an den CD4 Teil entweder N- oder C-terminal fusioniert ist.

5. Ein Fusionspeptid gemäß Anspruch 2, worin alle oder ein Teil der ersten 177 Aminosäure-Reste des CD4 Moleküle an ihrem C-terminalen Ende mit dem N-terminalen Ende der Peptid-Sequenz, die von den Glycophorin-bindenden Peptid 130 Aminosäure-Resten 201-774 abstammt, verbunden sind. Die resultierende Peptid-Sequenz ist

NH2-CD4-GBP 130-COOH
1-177 201-774.

6. Ein Fusionspeptid gemäß Anspruch 2, worin die gesamte oder ein Teil der Peptid-Sequenz, die Aminosäuren 1-177 des CD4 Moleküls umfasst, an dem C-terminalen Ende mit dem N-terminalen Ende der gesamten oder eines Teils der Peptid-Sequenz, die von den Aminosäuren 1-774 des Glycophorin-bindenden Peptide 130 abstammt, fusioniert ist

NH2-CD4—GBP 130-COOH
1-177   1-774.

7. Ein Fusionspeptid gemäß Anspruch 2, umfassend die von CD4 abstammende Peptid-Sequenz, alle oder einen Teil der Aminosäure-Reste 1-177 von CD4, die an ihrem C-terminalen Ende mit dem N-terminalen Ende der Reste 70-427 vom Glycophorin-bindenden Peptid-Homolog GBPH fusioniert ist

NH2-CD4—GBPH-COOH
1-177   70-427.

8. Ein Fusionspeptid gemäß Anspruch 2, umfassend die von CD4 abstammende Peptid-Sequenz 1-371, insgesamt oder zum Teil an ihrem C-terminalen Ende mit dem N-terminalen Ende von allen oder einem Teil der Reste 70-427 vom Glycophorin-bindenden Peptid-Homolog GBPH fusioniert ist,

NH2-CD4—GBPH-COOH
1-377    70-427.

9. Ein Fusionspeptid gemäß Anspruch 2, umfassend die Aminosäure-Reste 1-371 der vom menschlichen CD4 abstammenden Peptid-Sequenz, die alle oder zum Teil an ihrem C-terminalen Ende mit dem N-terminalen Ende der Aminosäure-Reste 109-427 der Glycophorin-bindenden Protein-Homolog GBPH Sequenz fusioniert sind

NH2-CD4—GBPH-COOH

1-371    109-427.

10. Ein Fusionspeptid gemäß Anspruch 2, das aus der Fusion von Peptid-Sequenzen gebildet ist, die von allen oder zum Teil von den Aminosäure-Resten 1-371 des CD4 Moleküle abstammen und an ihrem C-terminalen Ende mit dem N-terminalen Ende von EBA 175, Erythrocyten-bindendes Antigen 175, ein Malaria-Peptid, Reste 20-1435, alle oder zum Teil und insbesondere der Teil, der die Aminosäure-Reste 1062-1103 von EBA175 enthält und auch als EBA Peptid4 bezeichnet ist, fusioniert sind; wobei die allgemeine Formel lautet:

NH2-CD4—EBA175-COOH
1-371    20-1435.

11. Ein Fusionspeptid gemäß einem der Ansprüche 2 bis 10, worin die CD4 Komponente am N-terminalen Ende mit dem C-terminalen Ende der von Malaria abstammenden Peptid-Komponente fusioniert ist.

12. Ein Fusionspeptid gemäß Anspruch 2, das durch die Fusion eines Peptid-Segments gebildet ist, das von den Plasmodium vivax Duffy Rezeptor-Molekülen abstammt und alle oder einen Teil der Aminosäure-Reste 23-1051 des Peptid-Segments am C-terminalen Ende mit dem C-terminalen Ende der Peptid-Segmente verbunden sind, die von dem CD4 Molekül abstammen und alle oder ein Teil der Peptid-Segmente die Aminosäure-Reste 1-371 von CD4 umfassen; wobei die besagte Fusion oder Verbindung durch Verbinden von Aminosäure-Segmenten an nicht spezifizierten Stellen durch Disulfid-Bindungen mit Hilfe von Vernetzungsmitteln beliebigen Typs erfolgt;

NH2—P Vivax—IC CI————CD4—NH2
Duffy
23-1051                    371-1
— J————
nicht spezifiziertes Verbindungssegment .

13. Ein Fusionspeptid gemäß Anspruch 2, das durch die Fusion von Peptid-Segmenten gebildet ist, die von dem Pf200 oder PMMSA Malaria-Parasiten Molekül abstammen und ganz oder zum Teil an dem C-terminalen Ende mit einem Peptid verbunden sind, das von CD4 abstammt und die Aminosäure-Reste 371-1 umfasst; wobei die besagte Fusion oder Verbindung durch Verbinden von Aminosäure-Segmenten an nicht spezifizierten Stellen durch Disulfid-Bindungen mit Hilfe von Vernetzungsmitteln beliebigen Typs erfolgt:

NH2—PMMSA—C C————CD4-NH2
Peptid-                    Peptid-
Segment,                   Segment
codiert von                codiert von
Nucleinsäure               Nucleinsäure
175-5169                   1260-114
—— J————
Verbindungsarm.

14. Ein Fusionspeptid, das aus einem Segment des P Knowlesi Duffy Rezeptor-Moleküls, das an seinem C-terminalen Ende mit dem C-terminalen Ende vom CD4 Peptid verbunden ist, das alle oder einen Teil der Aminosäure-Reste 371-1 von CD4 umfasst, gebildet ist; wobei die besagte Fusion oder Verbindung durch Verbinden von Aminosäure-Segmenten an nicht spezifizierten Stellen durch Disulfid-Bindungen mit Hilfe von Vernetzungsmitteln beliebigen Typs erfolgt:

NH2—P Knowlesi—C    C————————CD4—NH2

Duffy                                    371-1

—— J——————————

15. Ein Fusionspeptid gemäß einem der Ansprüche 12, 13 und 14, worin die von CD4 abstammende Komponente N-terminal mit dem C-terminalen Ende des vom Merozoiten abstammenden Peptid-Pragments verbunden ist.

16. Ein Fusionspeptid gemäß einem der Ansprüche 12, 13 und 14, worin das vom Merozoiten abstammende Peptid-Fragment N-terminal mit dem C-terminalen Ende des CD4 Peptid-Fragments verbunden ist.

17. Ein Fusionspeptid gemäß einem der Ansprüche 2 bis 16, worin zwischen dem von CD4 abstammenden Peptid und dem vom Merozoiten abstammenden Peptid ein Peptid-Fragment positioniert ist, das die gesamte oder einen Teil der folgenden Aminosäure-Sequenz umfasst:
S T K G P S V F P L A P S S K S T S G G T A A L G C L V K
D Y F P E P V T V S Y N S G A L T S G V H T F P A V L Q S
S G L Y S L S S V V T V P S S S L G T Q T Y I C N V N H K
P S N T L V D K K V E P K S C D K T H T C P P C P A P E L
L G G P S V F L F P P K P K D T L M I S R T P E V T C V V
V D V S H E D P E V K F N W Y V D G V E V H N A K T K P R
E Q Y N S T Y R V V S V L T V L H Q D W L N G K E Y K C K
V S N K A L P A P I E K T I S K A K G Q P R E P Q V Y T L
P P S R D E L Y K N Q V S L T C V K G F Y P S D I A V E W
E S N G Q P E N N Y K T T P P V L D S D G S F F L Y S K L
T V D K S R W Q Q G N V F S C S V M H E A L H N G Y T Q K
S L S L S P G K.

18. Ein Protein-Gen, das ein Fusionspeptid gemäß einem der vorausgehenden Ansprüche codiert.

19. Ein Fusionspeptid gemäß einem der Ansprüche 1 bis 17 zur Verwendung
(a) bei der Behandlung einer HIV 1 oder HIV 2 Infektion; oder
(b) bei Prophylaxe der Übertragung von HIV 1 oder HIV 2; oder
(c) bei Verringerung viraler Titer von HIV 1 oder HIV 2 infizierten Personen; oder
(d) bei Verbesserung der Verwendbarkeit von Bluttransfusionen oder ihrer Sicherheit.

20. Ein Fusionspeptid gemäß einem der Ansprüche 1 bis 17 in Form von Augentropten.

21. Ein Fusionspeptid gemäß Anspruch 19 in Form eines gegen eine HIV 1 oder HIV 2 Infektion gerichteten Impfstoffs.

22. Die Verwendung eines Fusionspeptids gemäß einem der Ansprüche 1 bis 17 zur Herstellung eines Medikaments
(a) zur therapeutischen Verwendung als ein Empfängnisverhütungsmittel in Form von entweder Schaum, Creme, Spray, Gel oder verzögertes Freisetzungsmittel, das in einer empfängnisverhütenden Vorrichtung inkorporiert ist; oder
(b) zur Behandlung einer HIV 1 oder HIV 2 Infektion.

23. Verwendung gemäß Anspruch 22, worin das Medikament in Form eines gegen eine HIV 1 oder HIV 2 Infektion gerichteten Impfstoffs ist.

24. Verwendung eines Fusionspeptids gemäß einem der Ansprüche 1 bis 17 zur Herstellung eines Medikamente zur Prophylaxe oder Behandlung von Hepatitis B, C oder D.

25. Ein Test-Set zur beabsichtigter Verwendung für die Detektion einer HIV Infektion oder für die quantitative Bestimmung der Höhe der viralen Titer, wobei das Test-Set ein Fusionspeptid gemäß einem der Ansprüche 1 bis 17 umfasst.

## Revendications

1. Peptide de fusion hybride pour utilisation en thérapie formé par la fusion de deux ou plusieurs composants dans lesquels un composant est une séquence peptidique d'un variant de substitution, de délétion, d'insertion, de souche ou d'espèce d'une séquence peptidique ayant une affinité pour les globules rouges, dérivée d'un peptide du mérozoite parasitaire du paludisme, en particulier les segments du peptide de liaison à la glycophorine 130 (GBP130), ou les homologues du peptide de liaison à la glycophorine (GBPH), ou l'antigène de liaison aux erythrocites 175 (EBA175), ou le récepteur Duffy de plasmodium vivax ou l'antigène de surface pré majeur de mérozoite (PMMSA) ou l'antigène de surface du mérozoite de plasmodium falciparum (Pf200), fusionné à tout ou partie d'un segment de peptide se liant à un virus dérivé d'un récepteur viral ou un variant de liaison à un virus, par substitution, délétion ou insertion de celui-ci.

2. Peptide de fusion hybride selon la revendication 1 dans lequel le récepteur viral est l'un de :
(a) CD4 ;
(b) un fragment Fab d'immunoglobuline ayant une affinité pour les virus circulant dans le sang ; et
(c) un récepteur viral de liaison du virus de l'hépatite B, C ou D ou un anticorps se liant à un virus de l'hépatite.

3. Peptide de fusion selon la revendication 2 dans lequel l'extrémité C terminale de CD4 est jointe à l'extrémité N terminale du peptide de liaison à la glycophorine GBP130, le peptide de fusion résultant étant caractérisé en tant que :

NH2-CD4—GBP 130-COOH
1-371 201-774

4. Peptide de fusion selon la revendication 3 comprenant tout ou partie des résidus d'amino acide 1 à 371 de CD4 fusionnés à tout ou partie des résidus d'amino acide 201 à 774 de GBP130, la partie GBP130 étant fusionnée à la partie de CD4, soit par son extrémité N, soit par son extrémité C terminale.

5. Peptide de fusion selon la revendication 2 dans lequel les 117 premiers résidus d'amino acide, en toute ou partie, de la molécule CD4 sont joints par leur extrémité C terminale à l'extrémité N terminale de la séquence peptidique dérivée des résidus 201 à 774 du peptide de liaison à la glycophorine 130, la séquence peptidique résultante étant :

NH2-CD4—GBP 130-COOH
1-177 201-774

6. Peptide de fusion selon la revendication 2 dans lequel toute ou partie de la séquence peptidique comprenant les acides aminés 1 à 177 de la molécule CD4 est fusionnée par son extrémité C terminale a l'extrémité N terminale de toute ou partie de la séquence peptidique dérivée des acides aminés 1 à 174 du peptide de liaison à la glycophorine 130, la séquence peptidique résultante étant :

NH2-CD4—GBP 130-COOH
1-177 1-774

7. Peptide de fusion selon la revendication 2 comprenant la séquence peptidique dérivée de CD4, allant des résidus d'acides aminés 1 à 177 de CD4 en totalité ou en partie, fusionnée par son extrémité C terminale à l'extrémité N terminale des résidus 70 à 427 de l'homologue du peptide de liaison à la glycophorine GBPH, la séquence peptidique résultante étant :

NH2-CD4-GBPH-COOH
1-177 70-427

8. Peptide de fusion selon la revendication 2 comprenant la séquence peptidique, allant des résidus d'acides aminés 1 à 371 dérivée de CD4, en totalité ou en partie, fusionnée par son extrémité C terminale à l'extrémité N terminale de tout ou partie des résidus 70 à 424 de l'homologue du peptide de liaison à la glycophorine GBPH, la séquence peptidique résultante étant :

NH2-CD4-GBPH-COOH
1-371        70-427

9. Peptide de fusion selon la revendication 2 comprenant la séquence du peptide dérivée du CD4 humain allant des résidus d'acides aminés 1 à 371, en totalité ou en partie, fusionnée par son extrémité C terminale à l'extrémité N terminale des résidus d'acides aminés 109 à 427 de la séquence de l'homologue de la protéine de liaison à la glycophorine GBPH, la séquence peptidique résultante étant :

NH2-CD4—GBPH-COOH
1-371 109-427

10. Peptide de fusion selon la revendication 2 formé par la fusion des séquences peptidiques dérivées de tout ou partie de la molécule CD4 allant des résidus d'acides aminés 1 à 371, fusionnée par son extrémité C terminale à l'extrémité N terminale de EBA175, de l'antigène de liaison aux erythrocytes 175, du peptide du paludisme, allant des résidus 20 à 1435, en totalité ou en partie, et en particulier la partie contenant les résidus d'acides aminés 1062 à 1103 de EBA175 également désignée par peptide EBA-4, la formule générale étant :

NH2-CD4-EBA175-COOH
1-371        20-1435

11. Peptide de fusion selon l'une quelconque des revendications 2 à 10 dans lequel le composant CD4 est fusionné par son extrémité N terminale à l'extrémité C terminale du composant peptidique dérivé du paludisme.

12. Peptide de fusion selon la revendication 2 formé par la fusion d'un segment de peptide dérivé des molécules du récepteur Duffy de plasmodium vivax comprenant tout ou partie des résidus d'acides aminés 23 à 1051 du segment peptidique, joint par son extrémité C terminale à l'extrémité C terminale de tout ou partie des segments peptidiques dérivés de la molécule CD4 comprenant les résidus d'acides aminés 1 à 371 de CD4 ; ladite fusion ou jointure étant réalisée par jointure des segments d'acides aminés à des sites non spécifiés par des ponts disulfures ou par des agents réticulants d'un type quelconque, la formule générale étant :

NH2—P Vivax—IC CI————————CD4—NH2
Duffy
23-1051 371-1
—— J——————.
segment de jointure non spécifié

13. Peptide de fusion selon la revendication 2 formé par la fusion des segments peptidiques dérivés en tout ou partie de Pf2000 ou de la molécule parasitaire du paludisme PMMSA joints par l'extrémité C terminale à un peptide dérivé de CD4 comprenant les résidus d'acides aminés 371 à 1, ladite fusion ou jointure étant réalisée par jointure des segments d'acides aminés à des sites non spécifiés par des ponts disulfures ou par des agents réticulants d'un type quelconque, la séquence des peptides résultante étant :

NH2-PMMSA——C C————CD4—NH2
segment de peptide
codé par
acide nucléique
175-5169
segment de peptide
codé par l'
acide nucléique
1260-114
——J————
bras de liaison

14. Peptide de fusion formé d'un segment de la molécule du récepteur P Knowlesi Duffy, joint par son extrémité C terminale à l'extrémité C terminale de tout ou partie du peptide CD4 comprenant les résidus d'acides aminés 371 à 1 de CD4, ladite fusion ou jointure étant réalisée par jointure des segments d'acides aminés à des sites non spécifiés par des liaisons disulfures ou des agents réticulants d'un type quelconque, la séquence du peptide résultante étant :

NH2—P Knowlesi—C C——————CD4—NH2
Duffy 371-1
—— J——————.

15. Peptide de fusion selon l'une quelconque des revendications 12, 13 et 14 dans lequel le composant dérivé de CD4 est joint par son extrémité N terminale à l'extrémité C terminale du fragment de peptide dérivé du mérozoïte.

16. Peptide de fusion selon l'une quelconque des revendications 12, 13 et 14 dans lequel le fragment de peptide dérivé du mérozoïte est joint par son extrémité N terminale à l'extrémité C terminale du fragment du peptide CD4.

17. Peptide de fusion selon l'une quelconque des revendications 2 à 16 dans lequel il y a, interposé entre le peptide dérivé de CD4 et le peptide dérivé du mérozoïte, un fragment peptidique comprenant toute ou partie de la séquence d'acides aminés suivante :
S T K G P S V F P L A P S S K S T S G G T A A L G C L V K
D Y F P E P V T V S Y N S G A L T S G V H T F P A V L Q S
S G L Y S L S S V V T V P S S S L G T Q T Y I C N V N H K
P S N T L V D K K V E P K S C D K T H T C P P C P A P E L
L G G P S V F L F P P K P K D T L M I S R T P E V T C V V
V D V S H E D P E V K F N W Y V D G V E V H N A K T K P R
E Q Y N S T Y R V V S V L T V L H Q D W L N G K E Y K C K
V S N K A L P A P I E K T I S K A K G Q P R E P Q V Y T L
P P S R D E L Y K N Q V S L T C V K G F Y P S D I A V E W
E S N G Q P E N N Y K T T P P V L D S D G S F F L Y S K L
T V D K S R W Q Q G N V F S C S V M H E A L H N G Y T Q K
S L S L S P G K.

18. Gène de protéine codant pour un peptide de fusion selon l'une quelconque des revendications précédentes.

19. Peptide de fusion selon l'une quelconque des revendications 1 à 17 pour utilisation :
(a) dans le traitement des infections à HIV 1 ou HIV 2 ; ou
(b) la prévention de la transmission de HIV 1 ou HIV 2 ; ou
(c) l'abaissement de titres viraux chez les personnes infectées par HIV 1 ou HIV 2 ; ou
(d) l'amélioration de l'efficacité de transfusions sanguines ou leur sécurité.

20. Peptide de fusion selon l'une quelconque des revendications 1 à 17 sous forme de gouttes oculaires.

21. Peptide de fusion selon la revendication 19 sous forme d'un vaccin dirigé contre une infection par HIV 1 ou HIV 2.

22. Utilisation d'un peptide de fusion selon l'une quelconque des revendications 1 à 17 pour la fabrication d'un médicament :
(a) pour utilisation thérapeutique en tant qu'agent contraceptif sous forme d'une mousse, crème, pulvérisation, gelée ou agent à libération retardée incorporé dans un dispositif contraceptif ; ou
(b) pour le traitement d'infections à HIV 1 ou HIV 2.

23. Utilisation selon la revendication 22 dans laquelle le médicament est sous forme d'un vaccin dirigé contre une infection à HIV 1 ou HIV 2.

24. Utilisation d'un peptide de fusion selon l'une quelconque des revendications 1 à 17 pour la fabrication d'un médicament pour la prévention ou le traitement de l'hépatite B, C ou D.

25. Kit de test dans un but d'utilisation pour la détection d'infections à HIV ou la quantification de valeurs de titres viraux et comprenant un peptide de fusion selon l'une quelconque des revendications 1 à 17.
